(19) Europäisches Patentamt
European Patent Office
Office européen des brevets

(11) Veröffentlichungsnummer: **0 488 169 A1**

(12) **EUROPÄISCHE PATENTANMELDUNG**

(21) Anmeldenummer: **91120186.1**

(22) Anmeldetag: **26.11.91**

(51) Int. Cl.[5]: **C07C 323/30**, C07C 217/52, C07C 217/70, C07D 295/08, C07D 261/08, C07D 213/32, C07D 211/58, C07D 333/28, C07D 209/16, A61K 31/135, A61K 31/33

(30) Priorität: **28.11.90 DE 4037819**

(43) Veröffentlichungstag der Anmeldung:
**03.06.92 Patentblatt 92/23**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Hänel, Heinz, Dr.**
**Taunusstrasse 148**
**W-6370 Oberursel(DE)**
Erfinder: **Kirsch, Reinhard, Dr.**
**Johannesallee 18**
**W-6230 Frankfurt am Main(DE)**
Erfinder: **Kottmann, Hariolf, Dr.**
**Am alten Birnbaum 6**
**W-6238 Hofheim am Taunus(DE)**

(54) **Beta-Hydoxyethylamine mit 1,1-disubstituiertem Cyclopropylrest, Verfahren zu ihrer Herstellung und ihre Verwendung.**

(57)

Verbindungen I

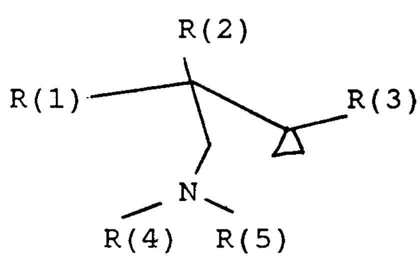

I

mit

R(1) gleich t-Butyl oder ein Aromat
R(2) gleich OH, Hal, Alkyl(Phenyl)carbonyloxy, Alkyloxy, Benzyloxy,
R(3) gleich (Cyclo)Alk(en)(in)yl oder ein Aromat,
R(4) gleich H, Alk(en)yl, Benzyl,
R(5) gleich H, (Cyclo)Alk(en)yl, Phenyl-Alk(en)yl oder R(4) und R(5) eine $(CH_2)_{4-6}$-Kette,
X gleich O, S, $SO_2$, SO,

sind antimykotisch wirksam, sie dienen ebenfalls zur Prophylaxe und zeigen Hemmung der Phospholipase von Candida albicans.

EP 0 488 169 A1

Die Erfindung betrifft β-Hydroxyethylamine mit 1,1-disubstituiertem Cyclopropylrest, ihre Herstellung und die Verwendung von β-Hydroxyethylaminen mit 1,1-disubstituiertem Cyclopropylrest und von solchen Verbindungen enthaltenden pharmazeutischen Zusammensetzungen als Pharmazeutika, insbesondere als Wachstumshemmer der pathogenen Phase amorpher Hefezellen, als Antimykotika und Pflanzenschutzmittel, z.B. Fungizide und Wachstumregulatoren, wobei die Verwendung der Verbindungen sowohl in der Prophylaxe als auch in der Therapie möglich ist.

Von einigen β-Hydroxyethylaminen, welche als Betablocker verwendet werden, ist bekannt, daß sie eine allerdings geringe Wirkung auf die Inhibierung der liposomalen Phospholipase A1 besitzen (vgl. K.Y. Hostetler et al., Biochemical Pharmacology 34, 521-524 (1985)). Wirkungsstärke und Verträglichkeit dieser Verbindungen sind jedoch nicht zufriedenstellend. (Beispiele sind z.B. Propranolol oder Metoprolol).

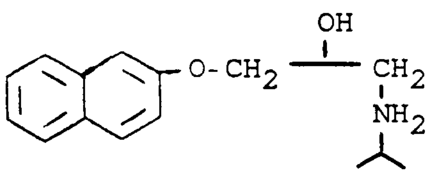

Propranolol

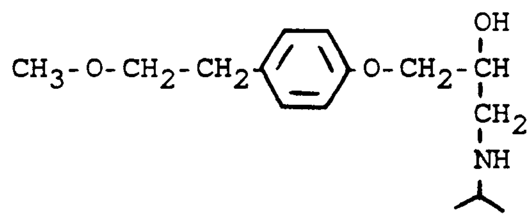

Metoprolol

Es wurde nun überraschenderweise gefunden, daß β-Hydroxyethylamine mit 1,1-disubstituiertem Cyclopropylrest, in Abhängigkeit von der Struktur der jeweiligen Substituenten, Hemmstoffe der Exoenzyme von Pilzen sind, fungizide sowie antimykotische Wirkung aufweisen und insbesondere hervorragende Wachstumshemmer der pathogenen Phase dimorpher Hefezellen sind.

Die Erfindung betrifft die Verbindungen der Formel I, deren Herstellung und deren Verwendung als Fungizide, als Antimykotika sowie als Wachstumshemmer der pathogenen Phase dimorpher Hefezellen zur Prophylaxe und Therapie von Pilzerkrankungen. Die Erfindung betrifft ferner diese Verbindungen enthaltende pharmazeutische Zusammensetzungen:

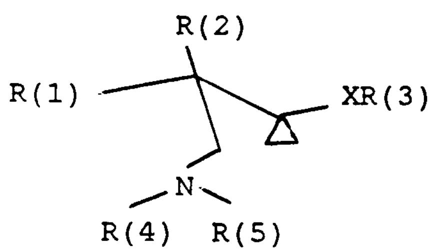

I

In der Formel I bedeuten:

R(1) t-Butyl, Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazolyl, Benzofuryl, Benzothienyl,
wobei die genannten Ringsysteme unsubstituiert oder mit 1-3 Substituenten, die gleich oder verschieden sind, substituiert sind, welche
F, Cl, Br, I, ($C_1$-$C_{18}$)-Alkyl (geradkettig oder verzweigt und unsubstituiert oder mit 1-9 F-, Cl-Atomen substituiert), ($C_3$-$C_{18}$)-Cycloalkyl [mono-, bi- oder multicyclisch, unsubstituiert oder ein- oder zweifach substituiert mit ($C_1$-$C_4$)-Alkyl (geradkettig oder verzweigt), ($C_1$-$C_4$)-Alkoxy - (geradkettig oder verzweigt), $CF_3$, F, Cl, Br, OH, wie z.B. Norbornyl-, Adamantyl, Dekahydronaphthyl] Y-($C_1$-$C_{18}$)-Alkyl (geradkettig oder verzweigt), Y-($C_3$-$C_{18}$)-Cycloalkyl [mono, bi- oder multicyclisch, unsubstituiert oder wie oben angegeben substituiert], ($C_2$-$C_{15}$)-Alkenyl (geradkettig oder verzweigt, ein- oder mehrfach ungesättigt), Y-($C_2$-$C_{15}$)-Alkenyl (geradkettig oder verzweigt, ein- oder mehrfach ungesättigt), Y-Phenyl, Y-($C_1$-$C_4$)-Alkyl-phenyl, Phenyl-($C_1$-$C_4$)-alkyl, Phenyl, CN, $NO_2$, $CO_2Z$ (mit Z gleich Phenyl, ($C_1$-$C_{15}$)-Alkyl, ($C_2$-$C_{15}$)-Alkenyl, wobei die aufgeführten Alkyl- und Alkenylsubstituenten geradkettig oder verzweigt sind und Phenyl unsubstituiert ist oder 1-3 Substituenten aus der Reihe F, Cl, $CF_3$, OH trägt), bedeuten,
mit Y gleich Sauerstoff, Schwefel, Sulfinyl, Sulfonyl,

R(2) OH, F, Cl, Br, ($C_1$-$C_{10}$)-Alkylcarbonyloxy (geradkettig oder verzweigt), ($C_1$-$C_{10}$)-Alkyloxy (geradkettig oder verzweigt), Benzyloxy (unsubstituiert oder 1-2fach substituiert mit F, Cl, Br, $CF_3$, $OCH_3$), Phenylcarbonyloxy, wobei der Phenylrest unsubstituiert ist oder mit 1-3 Substituenten aus der Reihe F, Cl, Br, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1-9fach substituiert durch F, Cl, Br, J und/oder $OCH_3$), ($C_2$-$C_{12}$)-Alkenyl (geradkettig oder verzweigt, ein- oder mehrfach ungesättigt, in Form des reinen E- oder Z-Diastereomeren oder als E/Z-Diastereomerengemisch, unsubstituiert oder 1- bis 9-fach substituiert durch F, Cl, Br, J und/oder $OCH_3$), ($C_2$-$C_{12}$)-Alkinyl (geradkettig oder verzweigt, mit einer oder mehreren Dreifachbindungen, unsubstituiert oder 1- bis 9-fach substituiert durch F, Cl, Br, J und/oder $OCH_3$), ($C_3$-$C_{12}$)-Alkeninyl (geradkettig oder verzweigt, eine oder mehrere Doppel- und/oder Dreifachbindungen enthaltend, in Form des reinen E- oder Z-Diastereomeren oder als E/Z-Diastereomerengemisch, unsubstituiert oder 1- bis 9-fach substituiert durch F, Cl, Br, J und/oder $OCH_3$), ($C_3$-$C_{12}$)-Cycloalkyl (unsubstituiert oder 1- bis 9-fach substituiert durch F, Cl, Br, J, $OCH_3$ und/oder $CH_3$), ($C_3$-$C_{12}$)-Cycloalkyl-($C_1$-$C_3$)-alkyl (unsubstituiert oder 1- bis 9-fach substituiert durch F, Cl, Br und/oder $CH_3$), Phenyl, Benzyl, Phenyl-($C_2$-$C_4$)-alkyl, Phenoxyphenyl, Phenylthiophenyl, Phenylsulfinylphenyl, Phenylsulfonylphenyl, Phenoxyphenyl($C_1$-$C_4$)-alkyl, Phenylthiophenyl-($C_1$-$C_4$)-alkyl, Naphthyl, Naphthyl-($C_1$-$C_4$)-alkyl, Biphenylyl, Biphenyl-($C_1$-$C_4$)-alkyl, Hetaryl, Hetaryl-($C_1$-$C_2$)-alkyl (mit Hetaryl gleich Thiophen, Furan, Pyridin, Oxazol, Isoxazol, Thiazol, Isthiazol, Pyrrol, 1,2,4-Oxadiazol, 1,3,4-Oxadiazol, Pyrimidin, Pyrazin, Benzothiofuran, Benzothiazol, Benzoxazol, Indol, Chinolin und Isochinolin), wobei die genannten aromatischen Ringsysteme und Hetarylsysteme unsubstituiert oder 1- bis 3-fach substituiert sind durch F, Cl, Br, $CF_3$, ($C_1$-$C_{10}$)-Alkyl (geradkettig oder verzweigt, unsubstituiert oder 1- bis 3-fach substituiert durch F, Cl und/oder $OCH_3$), ($C_3$-$C_6$)-Cycloalkyl (unsubstituiert), oder die aromatischen Ringsysteme oder die Hetarylsysteme substituiert sind durch Phenyl (unsubstituiert oder 1- bis 3-fach substituiert durch F, Cl, $OCH_3$), CN, OH, $NH_2$, NR(6)R(7), $NO_2$, Z'R(6), worin R(6) ($C_1$-$C_{10}$)-Alkyl (geradkettig oder verzweigt, unsubstituiert oder 1- bis 9-fach substituiert durch F, Cl und/oder $OCH_3$) und Z' gleich Sauerstoff oder Schwefel ist, oder R(6) ($C_3$-$C_{12}$)-Alkenyl (geradkettig oder verzweigt, ein- oder mehrfach ungesättigt, unsubstituiert oder 1- bis 3-fach substituiert durch F, Cl und/oder $OCH_3$) ist, oder R(6) Benzyl, Biphenylylmethyl, Naphthylmethyl, Phenyl, Thienylmethyl, Thienyl, ($C_1$-$C_{10}$)-Alkylcarbonyl (geradkettig oder verzweigt) oder Phenyl-carbonyl ist, wobei die hier für R(6) genannten Ringsysteme ihrerseits unsubstituiert oder 1- bis 3-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R(6) und R(7) gleich oder verschieden sein können,

R(4) H, ($C_1$-$C_{18}$)-Alkyl (geradkettig oder verzweigt), ($C_2$-$C_{10}$)-Alkenyl (geradkettig oder verzweigt, ein- oder mehrfach ungesättigt), Benzyl (unsubstituiert oder ein- oder mehrfach substituiert durch F, Cl, Br, $CF_3$, ($C_1$-$C_4$)-Alkyl (geradkettig oder verzweigt), $OCH_3$, O-Phenyl oder Phenyl),

R(5) H, ($C_1$-$C_{18}$)-Alkyl (geradkettig oder verzweigt), ($C_2$-$C_{18}$)-Alkenyl (geradkettig oder verzweigt, ein- oder mehrfach ungesättigt), ($C_3$-$C_{12}$)-Cycloalkyl, (mono-, bi- oder tricyclisch,wie z.B. Cyclohexyl, Norbornyl oder Adamantyl) ($C_1$-$C_8$)-Alkyl-oxy-($C_2$-$C_{10}$)-alkyl, Phenyl-($C_1$-$C_6$)-Alkyl (geradkettig oder verzweigt), Phenyl-oxy-($C_1$-$C_6$)-alkyl, Phenyl-thio-($C_1$-$C_6$)-alkyl, Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Thienyl, Thienyl-methyl, Phenyl, wobei die bezüglich R(5) genannten Phenyl- oder Thienylsysteme unsubstituiert oder ein- bis 3-fach substituiert sind durch Substituenten aus der Gruppe F, Cl, Br, ($C_1$-$C_{10}$)-Alkyl (geradkettig oder verzweigt), ($C_3$-$C_8$)-Cycloalkyl, OH, SH, ($C_1$-$C_{10}$)-Alkoxy (geradkettig oder verzweigt), Phenyl, Benzyl, Phenethyl, Thio-phenyl, $C_aF_{a+1}$ mit a = 1-6), -O $(CH_2)_{1-2}$O-,

oder

R(5) ist ein Piperidin-4-yl-Rest, der unsubstituiert ist oder durch 1 bis 4 Methylgruppen substituiert ist,

oder

R(5) ist ein Indol-3-yl-($C_1$-$C_4$)-Alkylrest (geradkettig oder verzweigt)

oder

R(4) mit R(5) eine Kette aus $(CH_2)_m$-Einheiten mit m = 4-6 bilden, die durch O, S oder N-Atom unterbrochen sein kann, wobei N als weiteren Bindungspartner ein H-Atom, eine $CH_3$, Phenyl-, Benzyl oder Phenethylgruppe trägt und der so gebildete Heterocyclus an den $CH_2$-Einheiten unsubstituiert ist oder 1- bis 4 $CH_3$-Gruppen als Substituenten trägt)

und

X Sauerstoff, Schwefel, Sulfinyl oder Sulfonyl,

und ihre Salze mit pharmazeutisch akzeptablen Säuren und in Form von physiologisch hydrolysierbaren und pharmazeutisch akzeptablen Derivaten.

Bevorzugt sind Verbindungen der Formel I, worin bedeuten:

R(1) Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Pyridyl,

wobei die genannten Ringsysteme unsubstituiert oder mit 1-3 Substituenten, die gleich oder verschieden sind, substituiert sind, welche

F, Cl, ($C_1$-$C_{12}$)-Alkyl (geradkettig oder verzweigt und unsubstituiert oder mit 1-6 F-, Cl-Atomen substituiert), ($C_3$-$C_{18}$)-Cycloalkyl [mono, bi- oder multicyclisch (unsubstituiert oder einfach substituiert mit ($C_1$-$C_4$)-Alkyl (geradkettig oder verzweigt), ($C_1$-$C_4$)-Alkoxy (geradkettig oder verzweigt), $CF_3$, F, Cl, OH), wie z.B. Norbornyl-, Adamantyl, Dekahydronaphthyl], Y-($C_1$-$C_{10}$)-Alkyl (geradkettig oder verzweigt), Y-($C_3$-$C_{10}$)-Cycloalkyl [mono-, bi- oder multicyclisch, unsubstituiert oder wie oben angegeben substituiert], ($C_2$-$C_{10}$)-Alkenyl (geradkettig oder verzweigt, ein- oder mehrfach ungesättigt), Y-($C_2$-$C_{10}$)-Alkenyl (geradkettig oder verzweigt, ein- oder mehrfach ungesättigt), Y-Phenyl, Y-($C_1$-$C_2$)-Alkyl-phenyl, Phenyl-($C_1$-$C_2$)-alkyl, Phenyl, wobei die aufgeführten Alkyl- und Alkenylsubstituenten geradkettig oder verzweigt sind und Phenyl unsubstituiert ist oder 1- bis 3 Substituenten aus der Reihe F, Cl, $CF_3$, OH trägt, bedeuten,

mit Y gleich Sauerstoff, Schwefel,

R(2) OH, F, Cl, ($C_1$-$C_{10}$)-Alkylcarbonyloxy (geradkettig oder verzweigt), ($C_1$-$C_{10}$)-Alkyloxy (geradkettig oder verzweigt), Benzyloxy (unsubstituiert oder 1- bis 2-fach substituiert mit F, Cl, $CF_3$, $OCH_3$), Phenylcarbonyloxy, wobei der Phenylrest unsubstituiert ist oder mit 1-3 Substituenten aus der Reihe F, Cl, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1- bis 6-fach substituiert durch F, Cl, Br und/oder $OCH_3$), ($C_2$-$C_{12}$)-Alkenyl (geradkettig oder verzweigt, ein- oder mehrfach ungesättigt, unsubstituiert oder 1-3-fach substituiert durch F, Cl und/oder $OCH_3$), ($C_2$-$C_{10}$)-Alkinyl (geradkettig oder verzweigt, mit einer oder zwei Dreifachbindungen und unsubstituiert), ($C_3$-$C_{10}$)-Alkeninyl (geradkettig oder verzweigt, eine oder mehrere Doppel- und/oder Dreifachbindungen enthaltend und unsubstituiert), ($C_3$-$C_{12}$)Cycloalkyl (unsubstituiert oder 1-3-fach substituiert durch F, Cl, Br und/oder $CH_3$), ($C_3$-$C_{12}$)-Cycloalkyl-($C_1$-$C_3$)-alkyl (unsubstituiert), Phenyl, Benzyl, Phenyl($C_2$-$C_3$)-alkyl, Phenoxyphenyl, Phenylthiophenyl, Phenylsulfinylphenyl, Phenylsulfonylphenyl, Phenoxyphenyl-($C_1$-$C_4$)alkyl, Phenylthiophenyl-($C_1$-$C_2$)-alkyl, Naphthyl, Naphthyl-($C_1$-$C_2$)-alkyl, Biphenylyl, Biphenylyl-($C_1$-$C_2$)-alkyl, Hetaryl, Hetaryl-($C_1$-$C_2$)-alkyl [mit Hetaryl gleich Thiophen, Furan, Pyridin, Oxazol, Isoxazol, Thiazol, Isthiazol, 1,3,4-Oxadiazol, Pyrimidin, Benzothiofuran, Benzothiazol, Benzoxazol, Chinolin und Isochinolin], wobei die genannten aromatischen Ringsysteme und Hetarylsysteme unsubstituiert oder 1- bis 2-fach substituiert sind durch F, Cl, $CF_3$, ($C_1$-$C_{10}$)-Alkyl (geradkettig oder verzweigt, unsubstituiert oder 1- bis 2-fach substituiert durch F und Cl), ($C_3$-$C_6$)Cycloalkyl (unsubstituiert) oder die aromatischen Ringsysteme oder die Hetarylsysteme substituiert sind durch CN, NR(6)R(7), Z'R(6), worin R(6) ($C_1$-$C_{10}$)-Alkyl (geradkettig oder verzweigt, unsubstituiert oder 1- bis 3-fach substituiert durch F, Cl und/oder $OCH_3$) und Z' Sauerstoff oder Schwefel bedeutet, oder R(6) ($C_3$-$C_{10}$)-Alkenyl (geradkettig oder verzweigt, ein- oder mehrfach ungesättigt, unsubstituiert oder 1- bis 3-fach substituiert durch F, Cl und/oder $OCH_3$) ist oder R(6) Benzyl, Phenyl, Thienylmethyl, Thienyl oder Phenyl-carbonyl bedeutet, wobei die hier für R(6) genannten Ringsysteme ihrerseits unsubstituiert oder 1- bis 2-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R(6) und R(7) gleich oder verschieden sein können,

R(4) H, ($C_1$-$C_{10}$)-Alkyl (geradkettig oder verzweigt), Benzyl (unsubstituiert oder ein- oder zweifach substituiert durch F, Cl, $CF_3$, ($C_1$-$C_4$)-Alkyl (geradkettig oder verzweigt), $OCH_3$, O-Phenyl oder Phenyl),

R(5) H, ($C_1$-$C_{16}$)-Alkyl (geradkettig oder verzweigt), ($C_2$-$C_{16}$)-Alkenyl (geradkettig oder verzweigt, ein- oder zweifach ungesättigt), ($C_3$-$C_{12}$)-Cycloalkyl, (mono-, bi- oder tricyclisch, wie z.B. Cyclohexyl, Norbornyl oder Adamantyl), ($C_1$-$C_8$)-Alkyl-oxy($C_2$-$C_8$)-alkyl, Phenyl-($C_1$-$C_6$)-Alkyl (geradkettig oder verzweigt), Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Phenyl, wobei die bezüglich R(5) genannten Phenylsysteme unsubstituiert oder ein- bis 3-fach substituiert sind durch Substituenten aus der Gruppe F, Cl, ($C_1$-$C_4$)-Alkyl (geradkettig oder verzweigt), ($C_3$-$C_8$)-Cycloalkyl, ($C_1$-

$C_{10}$)-Alkoxy (geradkettig oder verzweigt), Benzyl, Phenethyl, Thiophenyl, -O-($CH_2$)$_{1-2}$-O-, oder

R(5) ist 2,2,6,6-Tetramethylpiperidin-4-yl, oder Indol-3-yl-($C_1$-$C_4$)-alkyl,

X Sauerstoff, Schwefel, Sulfinyl oder Sulfonyl,

und ihre Salze mit pharmazeutisch akzeptablen Säuren und in Form von physiologisch hydrolisierbaren und pharmazeutisch akzeptablen Derivaten.

Besonders bevorzugt sind Verbindungen der Formel I, worin bedeuten:

R(1) Phenyl, Biphenylyl, Phenoxyphenyl, Benzylphenyl, Benzyloxyphenyl, Phenylthiophenyl, Naphthyl, 1,2,3,4-Tetrahydronaphthyl, Indanyl, Pyridyl, wobei die genannten Ringsysteme unsubstituiert oder mit 1-3 Substituenten, die gleich oder verschieden sind, substituiert sind, welche F, Cl, ($C_1$-$C_{10}$)-Alkyl (geradkettig oder verzweigt und unsubstituiert oder mit 1-3 F, Cl Atomen substituiert), ($C_3$-$C_{10}$)-Cycloalkyl (mono-, bi- oder multicyclisch und unsubstituiert, wie z.B. Norbornyl-, Adamantyl, Dekahydronaphthyl), Y-($C_1$-$C_{10}$)-Alkyl (geradkettig oder verzweigt), Y-($C_3$-$C_{10}$)-Cycloalkyl (mono-, bi- oder multicyclisch und unsubstituiert), ($C_2$-$C_{10}$)-Alkenyl (geradkettig oder verzweigt, ein- oder mehrfach ungesättigt), Y-($C_2$-$C_{10}$)-Alkenyl (geradkettig oder verzweigt, ein- oder mehrfach ungesättigt), Y-Phenyl, Benzyl, Phenyl, wobei die aufgeführten Alkyl- und Alkenylsubstituenten geradkettig oder verzweigt sind und Phenyl unsubstituiert ist oder 1- bis 3 Substituenten aus der Reihe F, Cl, $CF_3$, trägt, bedeuten, mit Y gleich Sauerstoff, Schwefel,

R(2) OH, F, ($C_1$-$C_6$)-Alkylcarbonyloxy (geradkettig oder verzweigt), ($C_1$-$C_6$)-Alkyloxy (geradkettig oder verzweigt), Benzyloxy (unsubstituiert oder 1-fach substituiert mit F, Cl, $CF_3$), Phenylcarbonyloxy, wobei der Phenylrest unsubstituiert ist oder mit 1-3 Substituenten aus der Reihe F, Cl, Br, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1-3-fach substituiert durch F, Cl und/oder $OCH_3$), ($C_2$-$C_{12}$)-Alkenyl (geradkettig oder verzweigt, ein- oder mehrfach ungesättigt und unsubstituiert), ($C_3$-$C_{12}$)-Cycloalkyl (unsubstituiert), ($C_3$-$C_{10}$)-Cycloalkyl-($C_1$-$C_3$)-alkyl (unsubstituiert), Phenyl, Benzyl, Phenoxyphenyl, Phenylthiophenyl, Phenoxyphenyl-($C_1$)-alkyl, Phenylthiophenyl-($C_1$)-alkyl, Naphthyl-($C_1$)-alkyl, Biphenylyl, Biphenylyl-($C_1$)-alkyl, Hetaryl, Hetarylmethyl (mit Hetaryl gleich Thiophen, Furan, Pyridin, Oxazol, Isoxazol, Thiazol, Isthiazol, Pyrimidin, Benzothiazol und Benzoxazol), wobei die genannten aromatischen Ringsysteme und Hetarylsysteme unsubstituiert oder 1- bis 2-fach substituiert sind durch F, Cl, $CF_3$, ($C_1$-$C_{10}$)-Alkyl (geradkettig oder verzweigt und unsubstituiert), ($C_3$-$C_6$)-Cycloalkyl (unsubstituiert) oder die aromatischen Ringsysteme oder die Hetarylsysteme substituiert sind durch CN, NR(6)R(7), Z'R-(6), worin R(6) ($C_1$-$C_{10}$)-Alkyl (geradkettig oder verzweigt und unsubstituiert) und Z' gleich Sauerstoff oder Schwefel bedeutet oder R(6) ($C_3$-$C_6$)-Alkenyl (geradkettig oder verzweigt, ein- oder mehrfach ungesättigt und unsubstituiert) oder R(6) Benzyl, Phenyl, Thienylmethyl oder Thienyl bedeutet, wobei die hier für R(6) genannten Ringsysteme ihrerseits unsubstituiert oder 1- bis 2-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R(6) und R(7) gleich oder verschieden sein können,

R(4) H, ($C_1$-$C_{10}$)-Alkyl (geradkettig oder verzweigt), Benzyl (unsubstituiert oder ein- oder zweifach substituiert durch F, Cl, $CF_3$, ($C_1$-$C_4$)-Alkyl (geradkettig oder verzweigt), $OCH_3$, O-Phenyl oder Phenyl),

R(5) H, ($C_1$-$C_{12}$)-Alkyl (geradkettig oder verzweigt), ($C_2$-$C_{12}$)-Alkenyl (geradkettig oder verzweigt, ein- oder zweifach ungesättigt), ($C_3$-$C_{12}$)-Cycloalkyl, (mono-, bi- oder tricyclisch, wie z.B. Cyclohexyl, Norbornyl oder Adamantyl), Phenyl-($C_1$-$C_6$)-Alkyl (geradkettig oder verzweigt), Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Phenyl, wobei die bezüglich R(5) genannten Phenylsysteme unsubstituiert oder ein- bis 3-fach substituiert sind durch Substituenten aus der Gruppe F, Cl, -($C_1$-$C_4$)-Alkyl (geradkettig oder verzweigt), ($C_3$-$C_6$)-Cycloalkyl, ($C_1$-$C_{10}$)-Alkoxy (geradkettig oder verzweigt), Benzyl, Phenethyl,

X Sauerstoff oder Schwefel,

und ihre Salze mit pharmazeutisch akzeptablen Säuren und in Form von physiologisch hydrolysierbaren und pharmazeutisch akzeptablen Derivaten.

Ganz besonders bevorzugt sind Verbindungen der Formel I, worin bedeuten:

R(1) Phenyl, Biphenylyl, Phenoxyphenyl, Benzylphenyl, Benzyloxyphenyl, Phenylthiophenyl, Naphthyl, Pyridyl,

wobei die genannten Ringsysteme unsubstituiert oder mit 1-2 Substituenten, die gleich oder verschieden sind, substituiert sind, welche F, Cl, ($C_1$-$C_{10}$)-Alkyl (geradkettig oder verzweigt und unsubstituiert), ($C_3$-$C_{10}$)-Cycloalkyl (mono-, bi- oder multicyclisch und unsubstituiert, wie z.B. Norbornyl-, Adamantyl, Dekahydronaphthyl), Y-($C_1$-$C_{10}$)-Alkyl (geradkettig oder verzweigt), Y-($C_3$-$C_{10}$)-Cycloalkyl (mono-, bi- oder multicyclisch und unsubstituiert), ($C_2$-$C_{10}$)-Alkenyl (geradkettig oder verzweigt, ein- oder zweifach ungesättigt), Y-($C_2$-$C_{10}$)-Alkenyl (geradkettig oder verzweigt, ein- oder zweifach ungesättigt), Y-Phenyl, Benzyl, Phenyl, wobei die aufgeführten Alkyl- und Alkenylsubstituenten geradkettig oder verzweigt sind und Phenyl unsubstituiert ist oder 1 bis 2 Substituenten aus der Reihe F, Cl, $CF_3$ trägt), bedeuten, mit Y gleich Sauerstoff, Schwefel,

R(3) ($C_1$-$C_{10}$)-Alkyl (geradkettig oder verzweigt, unsubstituiert), ($C_3$-$C_{12}$)-Alkenyl (geradkettig oder verzweigt, ein- oder zweifach ungesättigt und unsubstituiert), ($C_3$-$C_6$)-Cycloalkyl (unsubstituiert), Phenyl, Benzyl, Phenoxyphenyl, Phenylthiophenyl, Phenoxyphenyl-($C_1$)-alkyl, Phenylthiophenyl-($C_1$)-alkyl, Naphthyl-($C_1$)-alkyl, Biphenylyl, Biphenylyl- ($C_1$)-alkyl, Hetaryl, Hetarylmethyl (mit Hetaryl gleich Thiophen, Pyridin, Oxazol, Isoxazol), wobei die genannten aromatischen Ringsysteme und Hetarylsysteme unsubstituiert oder 1-bis 2-fach substituiert sind durch F, Cl, $CF_3$, ($C_1$-$C_{10}$)-Alkyl (geradkettig oder verzweigt und unsubstituiert), ($C_3$-$C_6$)-Cycloalkyl (unsubstituiert) oder die aromatischen Ringsysteme oder die Hetarylsysteme unsubstituiert oder substituiert sind durch CN, NR(6)R(7), Z'R(6), worin R(6) ($C_1$-$C_{10}$)-Alkyl (geradkettig oder verzweigt und unsubstituiert) und Z' Sauerstoff oder Schwefel bedeutet oder R(6) ($C_3$-$C_6$)-Alkenyl (geradkettig oder verzweigt, ein- oder mehrfach ungesättigt und unsubstituiert) oder R(6) Benzyl oder Phenyl, bedeutet, wobei die hier für R(6) genannten Ringsysteme ihrerseits unsubstituiert oder 1- bis 2-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist,

R(4) H

R(5) H, ($C_1$-$C_{10}$)-Alkyl (geradkettig oder verzweigt), ($C_2$-$C_{10}$)-Alkenyl (geradkettig oder verzweigt, ein- oder zweifach ungesättigt), ($C_3$-$C_{12}$)-Cycloalkyl, (mono-, bi- oder tricyclisch,wie z.B. Cyclohexyl, Norbornyl oder Adamantyl), Phenyl-($C_1$-$C_6$)-Alkyl (geradkettig oder verzweigt), Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Phenyl, wobei die bezüglich R(5) genannten Phenylsysteme unsubstituiert oder ein- bis 2-fach substituiert sind durch Substituenten aus der Gruppe F, Cl, ($C_1$-$C_4$)-Alkyl (geradkettig oder verzweigt), ($C_3$-$C_6$)-Cycloalkyl, ($C_1$-$C_4$)-Alkoxy (geradkettig oder verzweigt), Benzyl, Phenethyl, und

X Sauerstoff oder Schwefel,

und ihre Salze mit pharmazeutisch akzeptablen Säuren und in Form von physiologisch hydrolysierbaren und pharmazeutisch akzeptablen Derivaten.

Als physiologisch hydrolysierbare und pharmazeutisch akzeptable Derivate sind beispielsweise an der Hydroxygruppe veresterte Derivate geeignet, die unter physiologischen Bedingungen zu den freien Säuren hydrolysierbar sind, die ihrerseits wieder physiologisch akzeptierbar sind, d.h. bei den notwendigen Dosen nicht toxisch sind.

Die Verbindungen (I) weisen mindestens ein asymmetrisches C-Atom auf und können daher als Enantiomere und Diastereomere auftreten. Die Erfindung umfaßt sowohl die reinen Isomeren als auch deren Gemische. Die Gemische von Diastereomeren können nach gebräuchlichen Methoden, zum Beispiel durch selektive Kristallisation aus geeigneten Lösungsmitteln oder durch Chromatographie an Kieselgel oder Aluminiumoxid in die Komponenten aufgetrennt werden.

Racemate können nach üblichen Methoden in die Enantiomeren aufgetrennt werden, so zum Beispiel durch Salzbildung mit einer optisch aktiven Säure, Trennung der diastereomeren Salze und Freisetzung der reinen Enantiomeren mittels einer Base.

Die Erfindung betrifft weiterhin ein Verfahren zur Herstellung von Verbindungen der Formel (I), dadurch gekennzeichnet, daß man eine Verbindung der Formel (II),

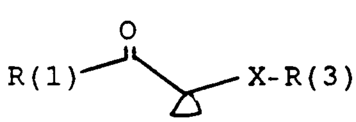

(II)

in welcher R(1), R(3) und X die genannten Bedeutungen haben, mit einem Schwefelylid der Formel III,

$$(H_3C)_2\underset{(O)_p}{\underset{\|}{S}}=CH_2 \qquad III,$$

worin p Null oder 1 bedeutet,
zu einer Verbindung der Formel IV

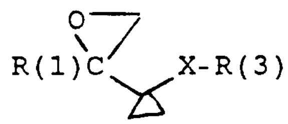

(IV)

umsetzt,
und anschließend die Verbindung IV mit einem Nucleophil der Formel MNR(4)R(5) umsetzt, in welcher R(4) und R(5) die genannten Bedeutungen hat und M Wasserstoff oder ein Metalläquivalent ist, wobei eine Verbindung I mit R(2) = OH entsteht, und falls gewünscht, diese Verbindung I acyliert oder alkyliert und gegebenenfalls am Schwefel einer Thioethergruppe zum Sulfoxid oder Sulfon oxidiert und gegebenenfalls die Verbindung I mit R(2) = OH in eine Verbindung mit R(2) = F, Cl oder Brom überführt und eine so erhaltene Verbindung der Formel (I) in Form der freien Base oder eines Säureadditionssalzes isoliert.

Zur Herstellung der Verbindungen der Formel (I) wird in einem ersten Reaktionsschritt zur Herstellung der Verbindungen der Formel (IV) ein Keton der Formel (II), in der R(1), R(3) und X die oben angegebenen Bedeutungen haben, mit einem Schwefelylid der Formel (III) in einem inerten Lösungsmittel, vorzugsweise Dimethylsulfoxid, oder in Gemischen von Dimethylsulfoxid mit anderen inerten Lösungsmitteln, z.B. Tetrahydrofuran, umgesetzt. Dabei ist bei Verwendung eines Schwefelylids der Formel (III), für das p = Null ist, ein Temperaturbereich zwischen -10° und 50°C, vorzugsweise zwischen 0° und 30°C zweckmäßig; bei Verwendung eines Schwefelylids der Formel (III), für das p = 1 ist, ein Temperaturbereich zwischen 0° und 80°, vorzugsweise zwischen 20° und 60°.

Die Umwandlung der Zwischenprodukte der Formel IV in die Endprodukte der Formel I mit R(2) = OH erfolgt in einem zweiten Reaktionsschritt durch Umsetzung mit einer Verbindung der Formel NR(4)R(5)-M, in der R(4),R(5) und M die oben angegebenen Bedeutungen haben.

Die vorgenannte Umsetzung erfolgt in einem Temperaturbereich von 20-160°C, gegebenenfalls in einem inerten Lösungsmittel, gegebenenfalls unter Verwendung einer Base.

Geeignete Lösungsmittel sind beispielsweise N,N-Dimethylformamid, N,N-Dimethylacetamid, Dimethylsulfoxid, Sulfolan, N-Methylpyrrolidon-2, Dioxan, Tetrahydrofuran, Acetonitril, 4-Methyl-2-pentanon, Methanol, Ethanol, Isopropylalkohol, Propanol, Butanol, Pentanol, Tert.-butylalkohol, Methylglykol, Methylenchlorid oder ein aromatischer Kohlenwasserstoff wie Benzol, Chlorbenzol, Nitrobenzol, Toluol oder Xylol oder Wasser. Es können auch Gemische der beispielhaft genannten Lösungsmittel verwendet werden.

Geeignete Basen sind beispielsweise Alkalimetall- oder Erdalkali-alkoholate oder -hydride wie z.B. Natriummethylat oder Natriumhydrid.

Die Umsetzung der Verbindungen der Formel II mit den Verbindungen der Formel III zu den Zwischenprodukten der Formel IV und Umsetzung dieser mit solchen der Formel MNR(3)R(4) zu Verbindungen der Formel I mit R(3) = OH kann auch im Eintopfverfahren durchgeführt werden.

Hierzu stellt man zunächst, wie oben angegeben, durch Umsetzung einer Verbindung der Formel II mit einer Verbindung der Formel III eine Lösung einer Verbindung der Formel IV in einem der oben angegebenen inerten Lösungsmittel her. Anschließend gibt man zu dieser Lösung eine mindestens äquivalente Menge einer Verbindung der Formel HNR(4)R(5).

Verbindungen der Formel I, in denen R(2) ($C_1$-$C_{10}$)-Alkoxy oder Benzyloxy (unsubstituiert oder ein oder zweifach mit F, Cl, Er, $CF_3$ oder $OCH_3$ substituiert) bedeutet, werden hergestellt, indem man eine Verbindung der Formel I mit R(2) = OH mit einem entsprechenden Alkyl- oder Benzylhalogenid, vorzugsweise einem Chlorid, Bromid, Tosylat oder Mesylat umsetzt, in Anwesenheit einer Base, in einem inerten Lösungsmittel, in einem Temperaturbereich von 0 - 100°C. Als Lösungsmittel werden vorzugsweise diejenigen verwendet, die zur Umsetzung von Verbindungen der Formel IV mit M-NR(3)R(4) angegeben sind, als Basen eignen sich z.B. die oben genannten anorganischen Basen oder organische Basen wie tert.-Amine wie Triethylamin, Ethylmorpholin oder z.B. DBU, Imidazol oder Pyridin.

Verbindungen der Formel I, in denen R(2) ($C_1$-$C_{10}$)-Alkylcarbonyloxy bedeutet, werden hergestellt,

indem man eine Verbindung der Formel I mit R(2) = H mit einem entsprechenden Alkylcarbonsäurehalogenid oder Phenylcarbonsäurehalogenid, vorzugsweise einem Alkylcarbonsäurechlorid, Alkylcarbonsäureanhydrid, Phenylcarbonsäurechlorid oder -anhydrid, umsetzt, in Gegenwart einer Base, in einem inerten Lösungsmittel, in einem Temperaturbereich von -20 bis 120 ° C.

Als Lösungsmittel und Basen werden vorzugsweise diejenigen verwendet, die zur Umsetzung von Verbindungen der Formel IV mit MNR(4)R(5) angegeben sind oder auch organische Basen, wie $NEt_3$ Pyridin oder DBU.

Verbindungen der Formel I, in denen R(2) F, Cl oder Brom bedeutet, werden hergestellt, indem man eine Verbindung der Formel I mit R(2) = OH mit Thionylchlorid, Thionylbromid, Schwefeltetrafluorid oder Diethylaminoschwefeltrifluorid ($Et_2NSF_3$) umsetzt, in einem inerten Lösungsmittel, gegebenenfalls in Gegenwart einer Base, in einem Temperaturbereich von (-40) - ( + 80) ° C.

Als Lösungsmittel und Basen werden vorzugsweise diejenigen verwendet, die zur Umsetzung von Verbindungen der Formel IV mit NMR(4)R(5) angegeben sind sowie z.B.Basen wie $NEt_3$, Ethylmorpholin, Pyridin oder DBU.

Verbindungen der Formel I, die eine Thioethergruppe enthalten, können am Schwefel zu einem Sulfoxid oder Sulfon oxidiert werden. Dazu werden solche Verbindungen der Formel I mit einem Oxidationsmittel wie z.B.

Wasserstoffperoxid oder m-Chlorperbenzoesäure umgesetzt, in einem inerten Lösungsmittel, in einem Temperaturbereich von -10 bis 80 ° C. Zur Herstellung von Sulfoxiden setzt man dazu ein Moläquivalent Oxidationsmittel ein, zur Herstellung von Sulfonen zwei Moläquivalente, gegebenenfalls auch einen Überschuß.

Als Lösungsmittel werden vorzugsweise diejenigen verwendet, die zur Umsetzung von Verbindungen der Formel IV mit M-NR(4)R(5) angegeben sind.

Herstellung der Ausgangsstoffe

Zur Herstellung von Verbindungen der Formel II, in denen R(1) und R(2) die angegebenen Bedeutungen haben, werden vorteilhaft nach den in den Patentanmeldungen DE-OS 36 08 792 DE-OS 36 08 727 angegebenen Verfahren angewendet.

Die Erfindung betrifft weiterhin ein Verfahren zur Herstellung der Verbindungen der Formel I, nach welchem man eine Verbindung der Formel II

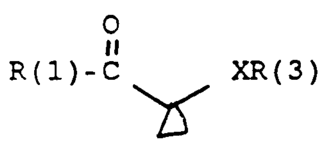

(II)

in welcher R(1), R(3) und X die genannten Bedeutungen haben,
mit einem Phosphorylid zu einer Verbindung der Formel V

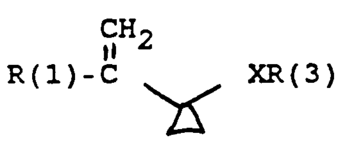

(V)

umsetzt, und anschließend die Verbindung V mit einem organischen Peroxid zu einer Verbindung der Formel IV

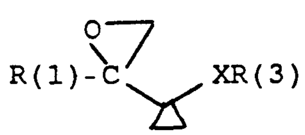

(IV)

umsetzt und IV wie oben bereits erwähnt weiter mit einem Nucleophil der Formel M-NR(4)R(5) umsetzt, in welcher R(4),R(5) und M die genannten Bedeutungen haben und M Wasserstoff oder ein Metalläquivalent

ist, wobei eine Verbindung I mit R(2) = OH entsteht und, falls gewünscht, diese Verbindung I acyliert, alkyliert oder in eine Verbindung mit R(2) = F, Cl oder Br, überführt, und gegebenenfalls am Schwefel einer Thioethergruppe zum Sulfoxid oder Sulfon oxidiert,
und gegebenenfalls in das Salz mit einer physiologisch verträglichen Säure überführt.

Zur Herstellung der Verbindungen der Formel I wird in einem ersten Reaktionsschritt zur Herstellung von Verbindungen der Formel V ein Keton der Formel II, in der R(1), R(3) und X die oben angegebenen Bedeutungen haben, mit einem Phosphorylid in einem inerten Lösungsmittel, in einem Temperaturbereich von -60 bis +80 °C umgesetzt.

Das dabei verwendete Phosphorylid wird durch Umsetzung eines Methyltriarylphosphoniumsalzes, vorzugsweise Methyltriphenylphosphonium-Chlorid, Bromid oder Jodid, mit einer starken Base in einem inerten Lösungsmittel hergestellt. Geeignete Basen sind z.B. Alkali- oder Erdalkalimetallhydride-, -amide oder -alkoholate, Natriumbistrimethylsilylamid oder Lithiumorganyle wie z.B. n-Butyl- oder Phenyl-Lithium.

Als Lösungsmittel werden diejenigen verwendet, die zur Umsetzung von Verbindungen der Formel IV mit M-NR(4)R(5) angegeben sind.

In einem zweiten Reaktionsschritt zur Herstellung von Verbindungen der Formel IV wird eine Verbindung der Formel V mit einem Oxidationsmittel umgesetzt, in einem inerten Lösungsmittel, in einem Temperaturbereich von -10 bis 80 °C, gegebenenfalls in Anwesenheit einer Base, einer Base und eines Nitrils wie z.B. Benzonitril, oder Thionyl-bis-imidazol oder -triazol.

Geeignete Oxidationsmittel sind z.B. $H_2O_2$ oder Percarbonsäuren, wie z.B. Peressigsäure, Trifluorperessigsäure, Perbenzoesäure oder m-Chlorperbenzoesäure.

Als Basen und Lösungsmittel werden diejenigen verwendet, die zur Umsetzung von Verbindungen der Formel IV mit M-NR(4)R(5) angegebenen sind.

In einem dritten Reaktionsschritt zur Herstellung von Verbindungen der Formel I mit R(2) = OH aus Verbindungen der Formel IV verfährt man wie im vorstehenden Verfahren angegeben.

Diese Verbindungen der Formel I mit R(2) = OH können, falls gewünscht, acyliert, alkyliert oder in eine Verbindung mit R(2) = F, Cl oder Er überführt, und gegebenenfalls am Schwefel einer Thioethergruppe zum Sulfoxid oder Sulfon oxidiert, und gegebenenfalls in das Salz mit einer physiologisch verträglichen Säure überführt werden, wie im vorstehenden Verfahren angegeben.

Die Verbindungen der Formel I, ihre Säureadditionssalze und ihre physiologisch hydrolysierbaren Derivate sind wertvolle Arzneimittel. Sie wirken insbesondere antimikrobiell und eignen sich zur Vorbeugung und Behandlung von Pilzinfektionen beim Menschen und bei verschiedenen Säugetierarten.

Nachfolgende Beispiele dienen zur Charakterisierung der Erfindung.

Beispiel 1

Darstellung einer Verbindung der Formel I durch Umsetzung eines Oxirans der Formel IV mit einem Amin ohne weiteres zusätzliches Lösungsmittel

Herstellung von

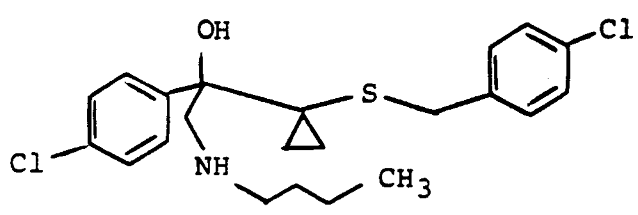

d.h.:

R(1) gleich 4-Chlorphenyl,
R(2) gleich OH,
R(3) gleich 4-Chlorbenzyl,
R(4) gleich Wasserstoff,
R(5) gleich n-Butyl und
X gleich Schwefel.

2.64 g Trimethylsulfoxoniumiodid (12 mmol, 1.2 Äquivalente) werden in 30 ml abs. DMS gelöst und portionsweise mit 0.39 g NaH (80 %ige Suspension in Paraffinöl, 13 mmol, 1.3 Äquivalente) versetzt und

das Reaktionsgemisch drei Stunden bei Zimmertemperatur bis zum Ende der Gasentwicklung gerührt. Anschließend werden zu dieser Lösung 3.37 g (10 mmol) des Ketons der Formel II mit R(1) gleich 4-Chlorphenyl und R(2) gleich 4-Chlorbenzyl und X gleich Schwefel, gelöst in 10 ml abs. DMSO, gegeben, und die Reaktionslösung wird vier Stunden bei Zimmertemperatur gerührt.

Zur Aufarbeitung wird die Reaktionslösung auf 200 ml Eiswasser gegossen und die wäßrige Phase viermal mit jeweils 100 ml Dichlormethan extrahiert. Die vereinigten organischen Phasen werden dreimal mit je 50 ml Wasser gewaschen, über $Na_2SO_4$ getrocknet, vom Trockenmittel abfiltriert und das Lösungsmittel im Vakuum entfernt.

Der ölige Rückstand besitzt laut $^1$H-NMR-Spektrum eine Struktur der Formel IV mit R(1) gleich 4-Chlorphenyl und R(2) gleich 4-Chlorbenzyl-thio und eine Reinheit von ca. 90 %:
Ausbeute: 3.52 g (hellgelbes Öl)
Dieses Zwischenprodukt wird anschließend mit 20 ml n-Butylamin versetzt und vier Stunden unter Rückfluß erhitzt. Der Verlauf der Reaktion wird dünnschichtchromatographisch verfolgt (Kieselgel, Laufmittel:Ethylacetat/Dichlormethan = 1/3).

Zur Aufarbeitung wird überschüssiges n-Butylamin am Rotationsverdampfer unter Vakuum entfernt und das Rohprodukt mittels Chromatographie über eine Kieselgelsäule (Laufmittel: Ethylacetat/Dichlormethan = 1/3) gereinigt. Ausbeute: 3,44 g (8,1 mmol, 81 % d.Th.), farbloses Öl

| Elementaranalyse $C_{22}H_{27}Cl_2NOS$: | | | | |
|---|---|---|---|---|
| C | gef.: | 62.4 | ber.: | 62.3 |
| H | gef.: | 6.3 | ber.: | 6.4 |
| N | gef.: | 3.2 | ber.: | 3.3 |

Beispiel 2

Darstellung einer Verbindung der Formel I durch Umsetzung eines Oxirans der Formel IV mit einem Amin im Autoklaven in DMSO als Lösungsmittel

Herstellung von

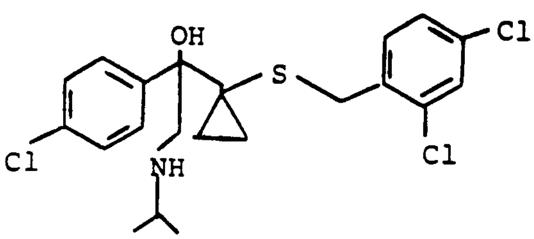

(1.19)

d.h.:

- R(1) gleich 4-Chlorphenyl,
- R(2 ) gleich OH,
- R(3) gleich 2,4-Dichlorbenzyl,
- R(4) gleich Wasserstoff,
- R(5) gleich Isopropyl und
- X gleich Schwefel.

3.08 g Trimethylsulfoxoniumiodid (14 mmol, 1 Äquivalent) werden in 35 ml abs. DMSO gelöst und portionsweise mit 0.46 g NaH (80 %ige Suspension in Paraffinöl, 11 mmol, 1.1 Äquivalente) versetzt und das Reaktionsgemisch vier Stunden bei Zimmertemperatur bis zum Ende der Gasentwicklung gerührt. Anschließend werden zu dieser Lösung 5.2 g (14 mmol) des Ketons der Formel II mit R(1) gleich 4-Chlorphenyl, R(2) gleich 2,4-Dichlorbenzyl und X gleich Schwefel, gelöst in 10 ml abs. DMSO, gegeben und die Reaktionslösung eine Stunde bei 40 °C und anschließend sechs Stunden bei Zimmertemperatur gerührt.

Zur Aufarbeitung wird die Reaktionslösung auf 200 ml Eiswasser gegossen und die wäßrige Phase viermal mit jeweils 100 ml Dichlormethan extrahiert. Die vereinigten organischen Phasen werden dreimal mit je 50 ml Wasser gewaschen, über $Na_2SO_4$ getrocknet, vom Trockenmittel abfiltriert und das Lösungsmittel im Vakuum entfernt.

Der ölige Rückstand besitzt laut $^1$H-NMR-Spektrum eine Struktur der Formel IV mit R(1) gleich 4-Chlorphenyl, R(2) gleich 2,4-Dichlorbenzyl und X gleich Schwefel sowie eine Reinheit von ca. 95 %.
Ausbeute: 2.97 g (hellgelbes Öl, 55 % d.Th.)

0.5 g des auf diese Weise hergestellten Oxirans (1.29 mmol) werden mit 3 ml Isopropylamin in 20 ml DMSO als Lösungsmittel zwei Stunden bei 140 ° C im Autoklaven umgesetzt.

Nach Beendigung der Reaktion wird überschüssiges Isopropylamin am Rotationsverdampfer unter reduziertem Druck entfernt und der verbleibende Rückstand mit Dichlormethan/Wasser extrahiert. Nach Trockung der organischen Phase mittels $Na_2SO_4$, Abtrennen des Trockenmittels und Entfernen des Lösungsmittels am Rotationsverdampfer verbleibt ein öliger Rückstand, der säulenchromatographisch gereinigt wird (Kieselgel; Laufmittel: Dichlormethan/Ethylacetat = 1/1). Man erhält ein farbloses Öl; das Produkt kristallisiert aus Diethylether aus.
Ausbeute: 370 mg (0.83 mmol, 65 % d.Th.)
Schmelzpunkt: 128 ° C

| Elementaranalyse $C_{21}H_{24}Cl_3NOS$: 444,85 g $mol^{-1}$ | | | | |
|---|---|---|---|---|
| C | gef.: | 56.5 | ber.: | 56.7 |
| H | gef.: | 5.4 | ber.: | 5.4 |
| N | gef.: | 3.2 | ber.: | 3.1 |

Beispiel 3

Darstellung einer Verbindung der Formel I durch Umsetzung eines Oxirans der Formel IV mit einem Amin in Ethanol/Wasser als Reaktionsmedium

Herstellung von

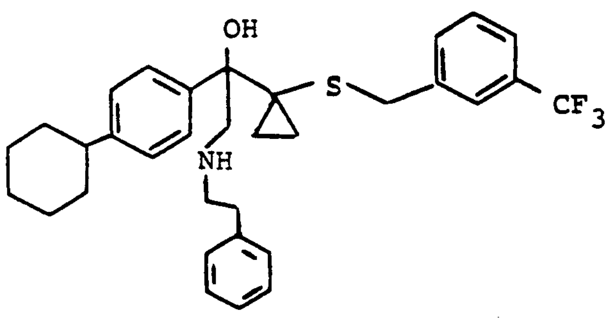

(1.25)

d.h.:

R(1) gleich 4-Cyclohexyl-phenyl,
R(2) gleich OH,
R(3) gleich 3-Trifluormethyl-benzyl,
R(4) gleich Wasserstoff,
R(5) gleich Phenylethyl und
X gleich Schwefel.

0.5 g (1.16 mmol) des auf die in Beispiel 1 beschriebene Weise hergestellten Oxirans der Formel II mit R(1) gleich 4-Cyclohexylphenyl, R(3) gleich 3-Trifluormethylbenzyl und X gleich Schwefel werden zusammen mit 1 ml Phenethylamin in 30 ml Ethanol und 2 ml Wasser gelöst und 5 Stunden bei 80 ° C gerührt; die Reaktion wird dünnschichtchromatographisch verfolgt (Kieselgel; Laufmittel: Dichlormethan).

Nach Beendigung der Umsetzung wird das Lösungsmittel am Rotationsverdampfer unter reduziertem Druck entfernt und der verbleibende Rückstand mit Dichlormethan/Wasser extrahiert. Nach Trocknung der organischen Phase mittels $Na_2SO_4$, Abtrennen des Trockenmittels und Entfernen des Extraktionsmittels am Rotationsverdampfer verbleibt ein öliger Rückstand, der säulenchromatographisch gereinigt wird (Kieselgel; Laufmittel: Dichlormethan). Man erhält ein farbloses Öl, welches aus Diethylether auskristallisiert: Ausbeute: 428 mg (0.77 mmol, 67 % d.Th.)

Schmelzpunkt: 81 ° C

| Elementaranalyse $C_{33}H_{38}F_3NOS$: 553.73 g $mol^{-1}$ | | | | |
|---|---|---|---|---|
| C | gef.: | 71.5 | ber.: | 71.6 |
| H | gef.: | 6.8 | ber.: | 6.9 |
| N | gef.: | 2.4 | ber.: | 2.5 |

Beispiel 4

Analog Beispielen 1, 2 oder 3, jedoch unter Verwendung entsprechender Verbindungen der Formel II und M-NR(4)R(5) können auch die in Tabelle 1 aufgeführten Verbindungen der Formel I mit R(2) gleich OH hergestellt werden. Beispiele 1, 2 und 3 sind ebenfalls in der Tabelle 1 enthalten.
Variante 1, 2 oder 3 kennzeichnet, ob gemäß Beispiel 1, 2 oder 3 verfahren wurde.

Beispiele 1.41-1.95 wurden gemäß Variante aus Beispiel 3 umgesetzt.

TABELLE 1

1.1

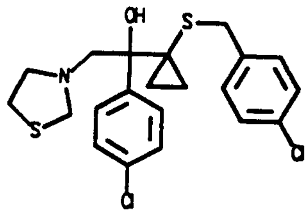

Fp.: Öl — Variante 1

| | ber.: | gef.: |
|---|---|---|
| C | 57.27% | 57.2 |
| H | 5.26% | 5.2 |
| Cl | 16.10% | |
| N | 3.18% | 3.2 |
| O | 3.63% | |
| S | 14.56% | |

1.2

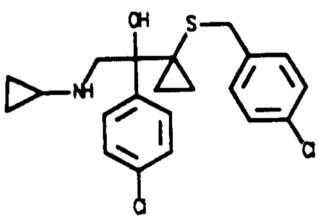

Fp.: Öl — Variante 3

| | ber.: | gef.: |
|---|---|---|
| C | 61.76% | 61.8 |
| H | 5.68% | 5.5 |
| Cl | 17.36% | |
| N | 3.43 | 3.3 |
| O | 3.92 | |
| S | 7.85 | |

1.3

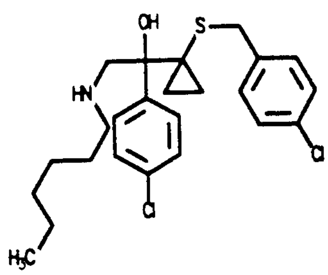

Fp.: Öl — Variante 1

| | ber.: | gef.: |
|---|---|---|
| C | 63.71% | 3.8 |
| H | 6.91% | 7.0 |
| Cl | 15.67% | |
| N | 3.10% | 7.0 |
| O | 3.54% | |
| S | 7.09% | |

1.4

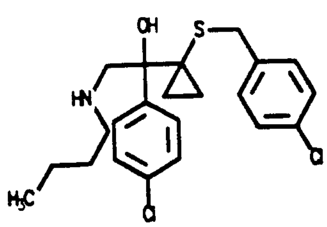

Fp.: Öl — Variante 1

| | ber.: | gef.: |
|---|---|---|
| C | 62.26% | 62.3 |
| H | 6.41 | 6.4 |
| Cl | 16.71% | |
| N | 3.30% | 3.4 |
| O | 3.77% | |
| S | 7.55% | |

Fortsetzung TABELLE 1

1.5

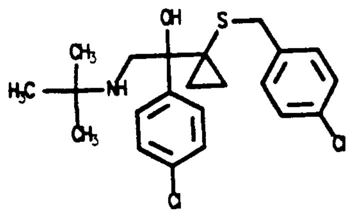

Fp.: Öl — Variante 1

| | ber.: | gef.: |
|---|---|---|
| C | 62.26% | 62.3 |
| H | 6.41% | 6.5 |
| Cl | 16.71% | |
| N | 3.30% | 3.3 |
| O | 3.77% | |
| S | 7.55% | |

1.6

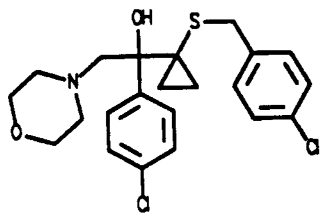

Fp.: Öl — Variante 1

| | ber.: | gef.: |
|---|---|---|
| C | 60.27% | 60.3 |
| H | 5.75% | 5.7 |
| Cl | 16.17% | |
| N | 3.19 | |
| O | 7.30 | 7.3 |
| S | 7.31 | |

1.7

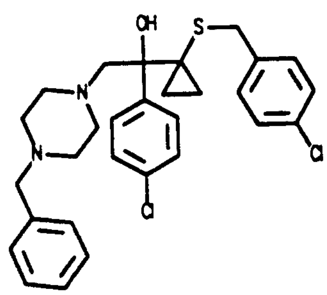

Fp.: Öl — Variante 2

| | ber.: | gef.: |
|---|---|---|
| C | 66.03% | |
| H | 6.11% | |
| Cl | 13.44% | |
| N | 5.31% | |
| O | 3.03% | |
| S | 6.08% | |

1.8

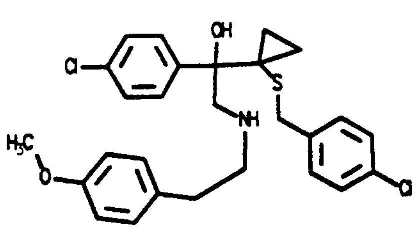

Fp.: Öl — Variante 3

| | ber.: | gef.: |
|---|---|---|
| C | 64.54% | 64.6 |
| H | 5.82 | 5.7 |
| Cl | 14.11% | |
| N | 2.79% | 2.9 |
| O | 6.37% | |
| S | 6.38% | |

Fortsetzung TABELLE 1

1.9

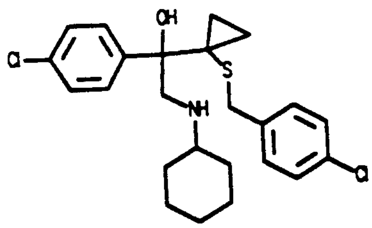

| Fp.: Öl | | Variante |
|---|---|---|
| ber.: | gef.: | 3 |
| C 63.99% | 63.9 | |
| H 6.49% | 6.4 | |
| Cl 15.74% | | |
| N 3.11% | 3.1 | |
| O 3.55% | | |
| S 7.12% | | |

1.10

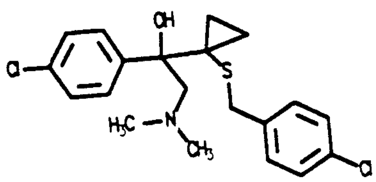

| Fp.: Öl | | Variante |
|---|---|---|
| ber.: | gef.: | 2 |
| C 60.60% | 60.6 | |
| H 5.75% | 5.8 | |
| Cl 17.89% | | |
| N 3.53% | 3.7 | |
| O 4.04 | | |
| S 8.09 | | |

1.11

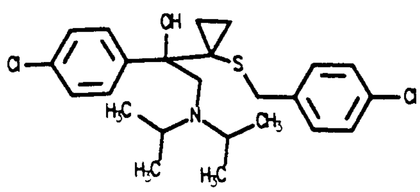

| Fp.: Öl | | Variante |
|---|---|---|
| ber.: | gef.: | 3 |
| C 63.71% | 63.8 | |
| H 6.91% | 6.8 | |
| Cl 15.67% | | |
| N 3.10% | 3.0 | |
| O 3.54% | | |
| S 7.09% | | |

1.12

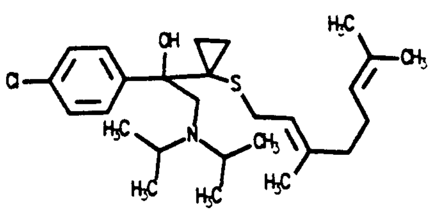

| Fp.: Öl | | Variante |
|---|---|---|
| ber.: | gef.: | 3 |
| C 69.87% | 69.9 | |
| H 9.12% | 9.0 | |
| Cl 7.64% | | |
| N 3.02% | 3.0 | |
| O 3.45% | | |
| S 6.91% | | |

Fortsetzung TABELLE 1

1.13

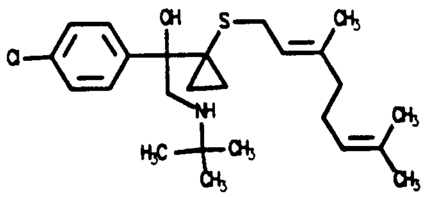

Fp.: Öl — Variante 3

| ber.: | | gef.: |
|---|---|---|
| C | 68.85% | 68.7 |
| H | 8.78% | 8.6 |
| Cl | 8.13% | |
| N | 3.21% | 3.1 |
| O | 3.67% | |
| S | 7.35% | |

1.14

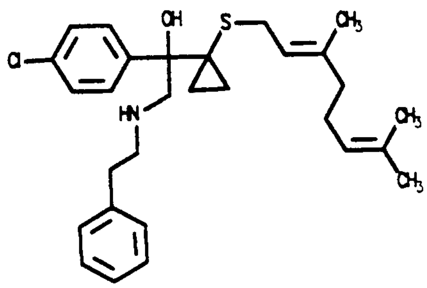

Fp.: Öl — Variante 2

| ber.: | | gef.: |
|---|---|---|
| C | 71.95% | 71.8 |
| H | 7.91% | |
| Cl | 7.32% | 7.2 |
| N | 2.89% | 2.9 |
| O | 3.30% | |
| S | 6.62% | |

1.15

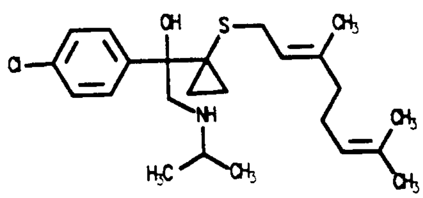

Fp.: Öl — Variante 3

| ber.: | | gef.: |
|---|---|---|
| C | 68.30% | 68.2 |
| H | 8.60% | 8.5 |
| Cl | 8.40% | |
| N | 3.32% | 3.2 |
| O | 3.79% | |
| S | 7.60% | |

1.16

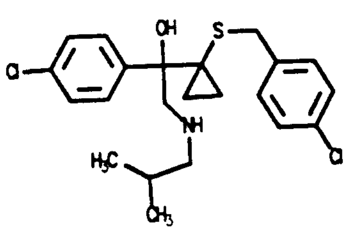

Fp.: Öl — Variante 3

| ber.: | | gef.: |
|---|---|---|
| C | 62.26% | 62.1 |
| H | 6.41% | 6.4 |
| Cl | 16.71% | |
| N | 3.30% | 3.2 |
| O | 3.77% | |
| S | 7.55% | |

Fortsetzung TABELLE 1

1.17

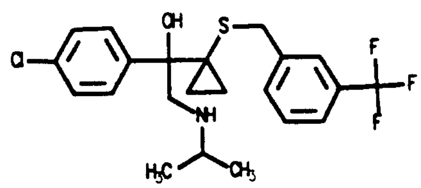

Fp.: Öl — Variante 2

| | ber.: | gef.: |
|---|---|---|
| C | 59.52% | 59.6 |
| H | 5.68% | 5.7 |
| Cl | 7.99% | |
| F | 12.84 | |
| N | 3.15% | 3.1 |
| O | 3.60% | |
| S | 7.22% | |

1.18

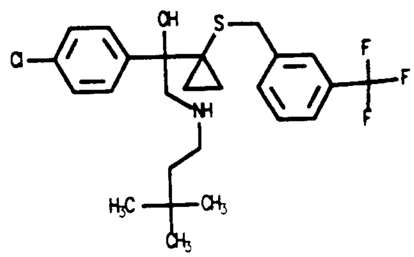

Fp.: Öl — Variante 3

| | ber.: | gef.: |
|---|---|---|
| C | 61.78% | 61.9 |
| H | 6.43% | 6.3 |
| Cl | 7.29% | |
| F | 11.73 | |
| N | 2.88% | 3.0 |
| O | 3.29% | |
| S | 6.60% | |

1.19

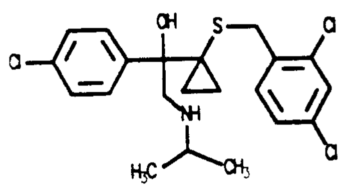

Fp.: Öl — Variante 2

| | ber.: | gef.: |
|---|---|---|
| C | 56.70% | 56.6 |
| H | 5.44% | 5.6 |
| Cl | 23.91% | |
| N | 3.15% | 3.1 |
| O | 3.60% | |
| S | 7.21% | |

1.20

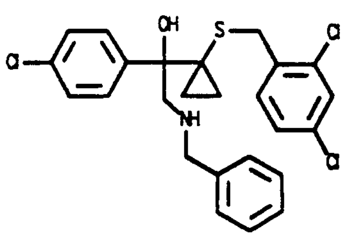

Fp.: Öl — Variante 2

| | ber.: | gef.: |
|---|---|---|
| C | 60.92% | 60.8 |
| H | 4.91% | 4.8 |
| Cl | 21.58% | |
| N | 2.84% | 2.7 |
| O | 3.25% | |
| S | 6.51% | |

Fortsetzung TABELLE 1

1.21

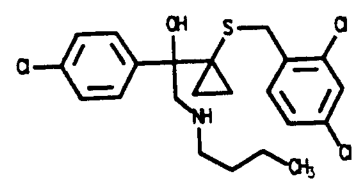

Fp.: Öl — Variante 3

| ber.: | | gef.: |
|---|---|---|
| C | 57.58% | 57.6 |
| H | 5.71% | 5.8 |
| Cl | 23.18% | |
| N | 3.05% | 3.1 |
| O | 3.49% | |
| S | 6.99% | |

1.22

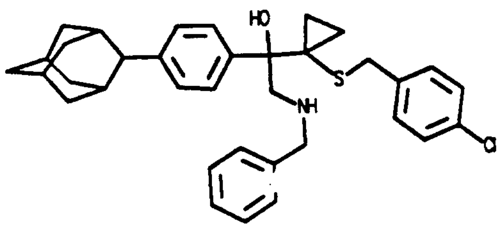

Fp.: Öl — Variante 3

| ber.: | | gef.: |
|---|---|---|
| C | 75.31% | 75.1 |
| H | 7.22% | 7.1 |
| Cl | 6.35% | |
| N | 2.51% | 2.4 |
| O | 2.87% | |
| S | 5.74% | |

1.23

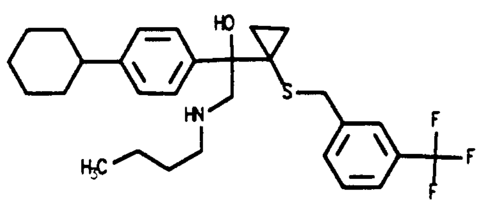

Fp.: Öl — Variante 2

| ber.: | | gef.: |
|---|---|---|
| C | 68.88% | 69.0 |
| H | 7.57% | 7.5 |
| F | 11.27% | |
| N | 2.77% | 2.6 |
| O | 3.16% | |
| S | 6.34% | |

1.24

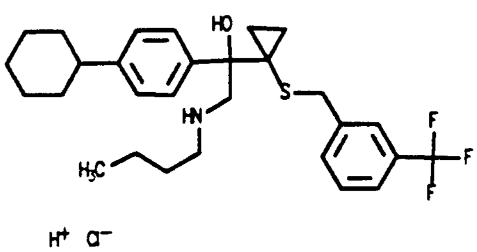

Fp.: Öl — Variante 3

| ber.: | | gef.: |
|---|---|---|
| C | 64.25% | 64.1 |
| H | 7.25% | 7.1 |
| Cl | 6.54% | |
| F | 10.51% | |
| N | 2.58% | 2.7 |
| O | 2.95% | |
| S | 5.91% | |

Fortsetzung TABELLE 1

1.25

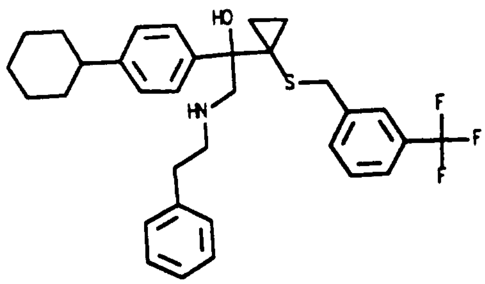

Fp.: Öl — Variante 3

| | ber.: | gef.: |
|---|---|---|
| C | 71.58% | 71.4 |
| H | 6.92% | 7.0 |
| F | 10.29% | |
| N | 2.53% | 2.5 |
| O | 2.89% | |
| S | 5.79% | |

1.26

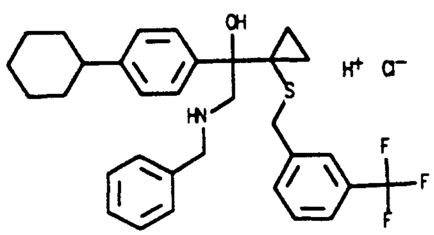

Fp.: Öl — Variante 3

| | ber.: | gef.: |
|---|---|---|
| C | 66.71% | 66.9 |
| H | 6.47% | 6.3 |
| Cl | 6.15% | |
| F | 9.89% | |
| N | 2.43% | 2.4 |
| O | 2.78% | |
| S | 5.57% | |

1.27

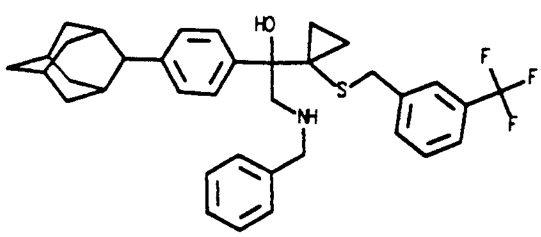

Fp.: Öl — Variante 3

| | ber.: | gef.: |
|---|---|---|
| C | 73.07% | 73.2 |
| H | 6.81% | 6.9 |
| F | 9.63% | |
| N | 2.37% | 2.2 |
| O | 2.70% | |
| S | 5.42% | |

1.28

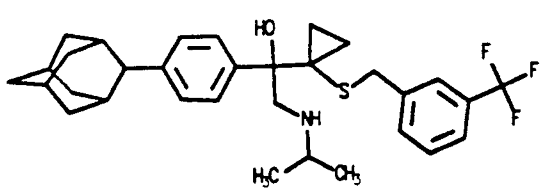

Fp.: Öl — Variante 3

| | ber.: | gef.: |
|---|---|---|
| C | 70.69% | 70.4 |
| H | 7.425% | 7.3 |
| F | 10.48% | |
| N | 2.58% | 2.6 |
| O | 2.94% | |
| S | 5.90% | |

Fortsetzung TABELLE 1

1.29

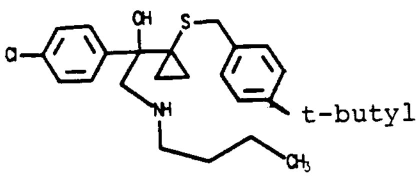

Fp.: Öl — Variante 3

| | ber.: | gef.: |
|---|---|---|
| C | 70.00% | 70.2 |
| H | 8.13% | 8.0 |
| Cl | 7.95% | |
| N | 3.14% | 2.5 |
| O | 3.59% | |
| S | 7.19% | |

1.30

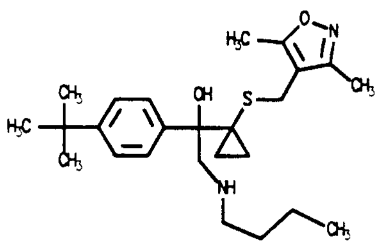

Fp.: Öl — Variante 3

| | ber.: | gef.: |
|---|---|---|
| C | 69.73% | 69.7 |
| H | 8.89% | 9.0 |
| N | 6.50% | 6.4 |
| O | 7.43% | |
| S | 7.45% | |

1.31

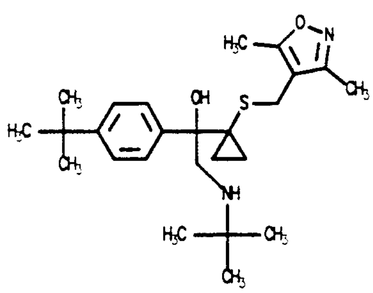

Fp.: Öl — Variante 3

| | ber.: | gef.: |
|---|---|---|
| C | 69.73% | 69.9 |
| H | 8.89% | 9.0 |
| N | 6.50% | 6.4 |
| O | 7.43% | |
| S | 7.45% | |

1.32

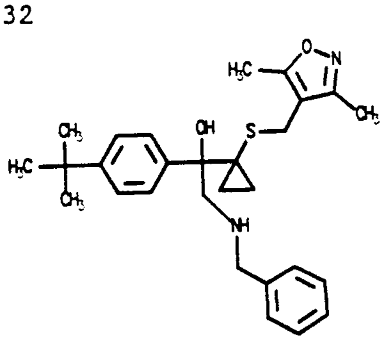

Fp.: Öl — Variante 3

| | ber.: | gef.: |
|---|---|---|
| C | 72.38% | 72.3 |
| H | 7.815% | 7.8 |
| N | 6.03% | 6.1 |
| O | 6.89% | |
| S | 6.90% | |

Fortsetzung TABELLE 1

1.33

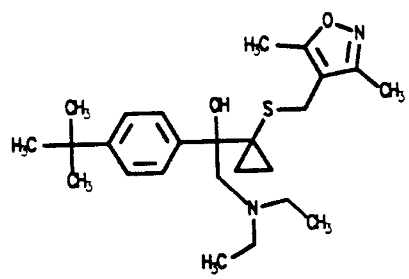

Fp.: Öl — Variante 3

| | ber.: | gef.: |
|---|---|---|
| C | 69.73% | 69.7 |
| H | 8.89% | 9.0 |
| N | 6.50% | 6.7 |
| O | 7.43% | |
| S | 7.45% | |

1.34

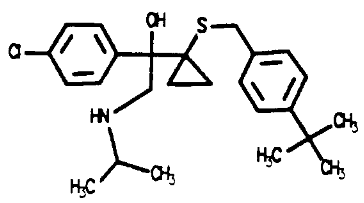

Fp.: Öl — Variante 3

| | ber.: | gef.: |
|---|---|---|
| C | 69.50% | 69.6 |
| H | 7.93% | 8.0 |
| Cl | 8.21% | |
| N | 3.24% | 3.1 |
| O | 3.70% | |
| S | 7.42% | |

1.35

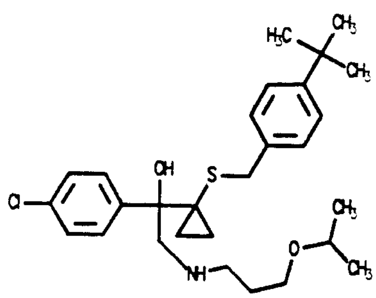

Fp.: Öl — Variante 3

| | ber.: | gef.: |
|---|---|---|
| C | 68.61% | 68.6 |
| H | 8.23% | 8.3 |
| Cl | 7.23% | |
| N | 2.86% | 2.7 |
| O | 6.53% | |
| S | 6.54% | |

1.36

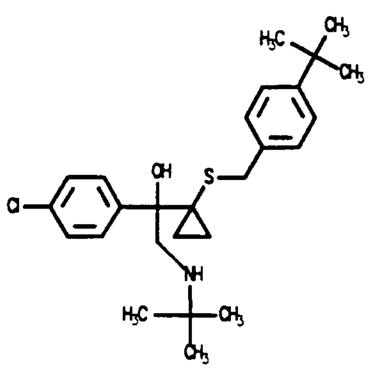

Fp.: Öl — Variante 3

| | ber.: | gef.: |
|---|---|---|
| C | 70.00% | 70.2 |
| H | 8.13% | 8.1 |
| Cl | 7.95% | |
| N | 3.14% | 3.1 |
| O | 3.59% | |
| S | 7.19% | |

Fortsetzung TABELLE 1

1.37

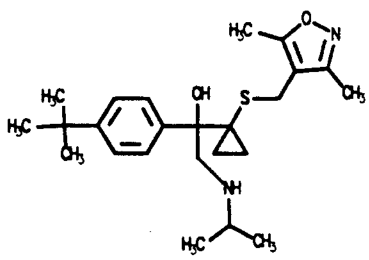

Fp.: Öl — Variante 3

| ber.: | | gef.: |
|---|---|---|
| C | 69.19% | 69.2 |
| H | 8.71% | 8.6 |
| N | 6.72% | 6.8 |
| O | 7.68% | |
| S | 7.70% | |

1.38

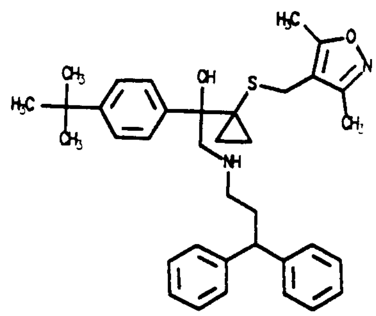

Fp.: Öl — Variante 3

| ber.: | | gef.: |
|---|---|---|
| C | 76.02% | 76.2 |
| H | 7.80% | 7.8 |
| N | 4.92% | 4.8 |
| O | 5.63% | |
| S | 5.64% | |

1.39

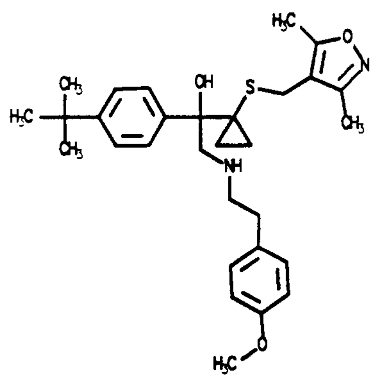

Fp.: Öl — Variante 3

| ber.: | | gef.: |
|---|---|---|
| C | 70.83% | 71.0 |
| H | 7.93% | 7.9 |
| N | 5.51% | 5.6 |
| O | 9.43% | |
| S | 6.40% | |

1.40

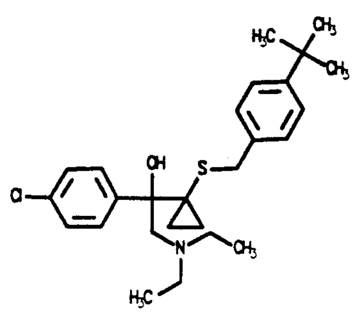

Fp.: Öl — Variante 3

| ber.: | | gef.: |
|---|---|---|
| C | 70.00% | 70.1 |
| H | 8.13% | 8.2 |
| Cl | 7.95% | |
| N | 3.14% | 3.2 |
| O | 3.59% | |
| S | 7.19% | |

1.41

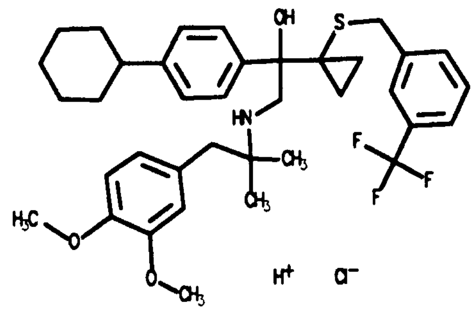

Fp.: 80°C

| ber.: | | gef.: | |
|---|---|---|---|
| C | 65.52% | C | 65.3 |
| H | 6.98% | H | 7.1 |
| Cl | 5.23% | N | 2.1 |
| F | 8.40% | | |
| N | 2.06% | | |
| O | 7.08% | | |
| S | 4.73% | | |

1.42

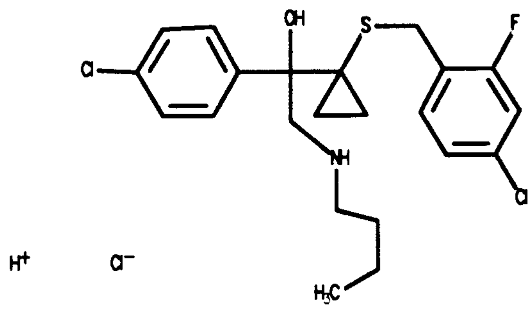

Fp.: Öl

| ber.: | | gef.: | |
|---|---|---|---|
| C | 55.18% | C | 55.1 |
| H | 5.68% | H | 5.6 |
| Cl | 22.21% | N | 2.9 |
| F | 3.97% | | |
| N | 2.92% | | |
| O | 3.34% | | |
| S | 6.70% | | |

1.43

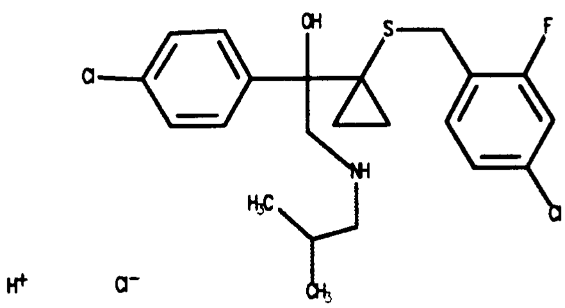

Fp.: 209-210°C

| ber.: | | gef.: | |
|---|---|---|---|
| C | 55.18% | C | 55.1 |
| H | 5.68% | H | 5.8 |
| Cl | 22.21% | N | 2.9 |
| F | 3.97% | | |
| N | 2.92% | | |
| O | 3.34% | | |
| S | 6.70% | | |

1.44

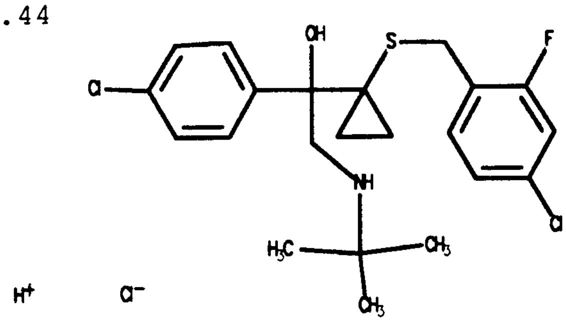

Fp.: 188-190°C

| ber.: | | gef.: | |
|---|---|---|---|
| C | 55.18% | C | 55.0 |
| H | 5.68% | H | 5.8 |
| Cl | 22.21% | N | 2.8 |
| F | 3.97% | | |
| N | 2.92% | | |
| O | 3.34% | | |
| S | 6.70% | | |

1.45

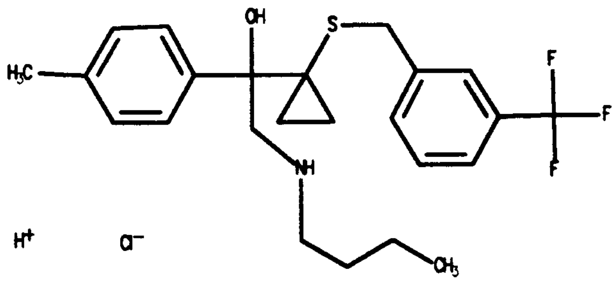

Fp.: 157-158°C

| ber.: | | gef.: | |
|---|---|---|---|
| C | 60.81% | C | 60.6 |
| H | 6.59% | H | 6.7 |
| Cl | 7.48% | N | 3.0 |
| F | 12.02% | | |
| N | 2.95% | | |
| O | 3.38% | | |
| S | 6.76% | | |

1.46

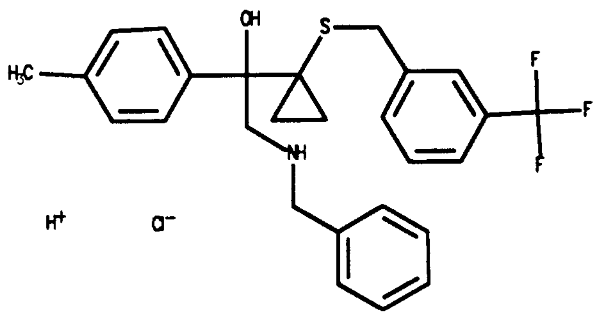

Fp.: 204°C

| ber.: | | gef.: | |
|---|---|---|---|
| C | 63.83% | C | 63.6 |
| H | 5.75% | H | 5.8 |
| Cl | 6.98% | N | 2.7 |
| F | 11.22% | | |
| N | 2.76% | | |
| O | 3.15% | | |
| S | 6.31% | | |

1.47

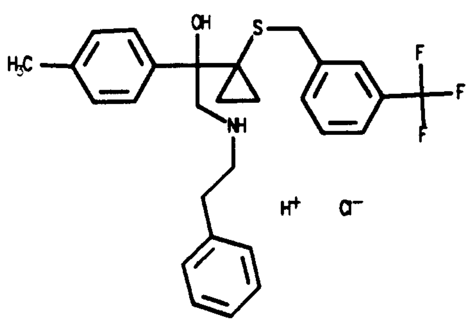

Fp.: 153°C

| ber.: | | gef.: | |
|---|---|---|---|
| C | 64.42% | C | 64.3 |
| H | 5.99% | H | 6.2 |
| Cl | 6.79% | N | 2.7 |
| F | 10.92% | | |
| N | 2.68% | | |
| O | 3.06% | | |
| S | 6.14% | | |

1.48

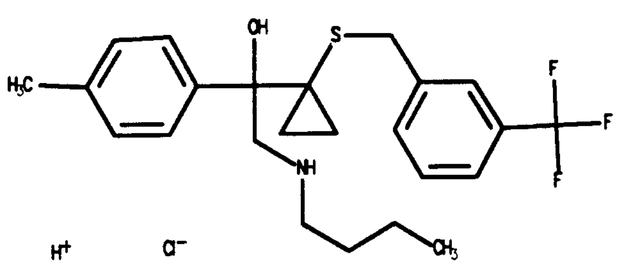

Fp.: 188°C

| ber.: | | gef.: | |
|---|---|---|---|
| C | 60.81% | C | 60.8 |
| H | 6.59% | H | 6.8 |
| Cl | 7.48% | N | 3.1 |
| F | 12.02% | | |
| N | 2.95% | | |
| O | 3.38% | | |
| S | 6.76% | | |

1.49

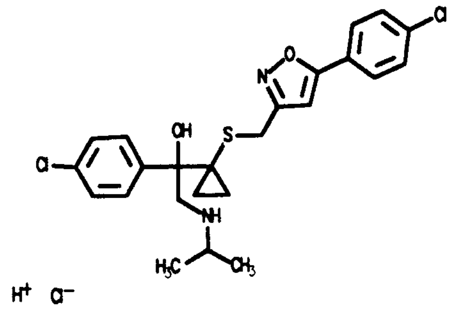

Fp.: 247°C

| ber.: | | gef.: | |
|---|---|---|---|
| C | 56.09% | C | 55.9 |
| H | 5.30% | H | 5.2 |
| Cl | 20.70% | N | 5.5 |
| N | 5.45% | | |
| O | 6.23% | | |
| S | 6.24% | | |

1.50

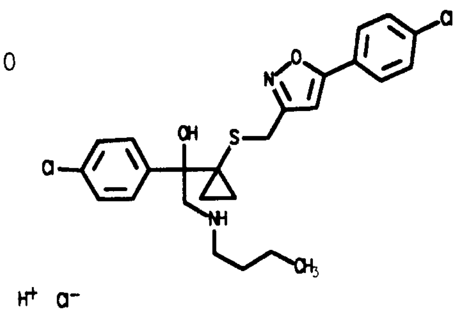

Fp.: 190°C

| ber.: | | gef.: | |
|---|---|---|---|
| C | 56.88% | C | 56.9 |
| H | 5.54% | H | 5.4 |
| Cl | 20.15% | N | 5.2 |
| N | 5.31% | | |
| O | 6.06% | | |
| S | 6.07% | | |

1.51

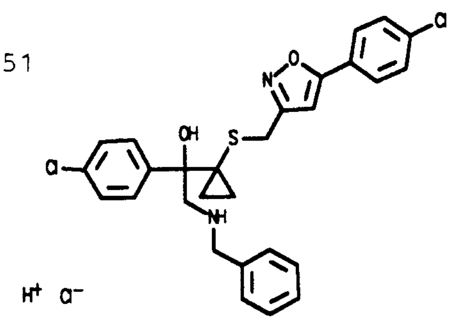

Fp.: 224°C

| ber.: | | gef.: | |
|---|---|---|---|
| C | 59.85% | C | 60.0 |
| H | 4.84% | H | 4.9 |
| Cl | 18.93% | N | 4.9 |
| N | 4.98% | | |
| O | 5.69% | | |
| S | 5.71% | | |

1.52

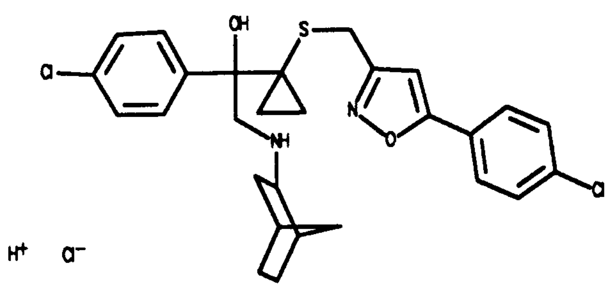

Fp.: 217°C

| ber.: | | gef.: | |
|---|---|---|---|
| C | 59.42% | C | 59.5 |
| H | 5.52% | H | 5.6 |
| Cl | 18.79% | N | 5.0 |
| N | 4.95% | | |
| O | 5.65% | | |
| S | 5.67% | | |

1.53

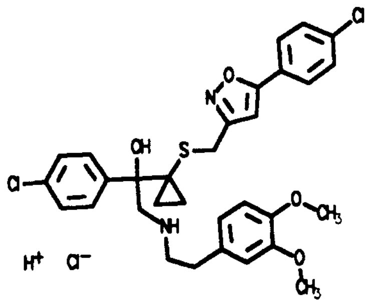

Fp.: 176°C

| ber.: | | gef.: | |
|---|---|---|---|
| C | 58.54% | C | 58.4 |
| H | 5.23% | H | 5.2 |
| Cl | 16.72% | N | 4.3 |
| N | 4.40% | | |
| O | 10.06% | | |
| S | 5.04% | | |

1.54

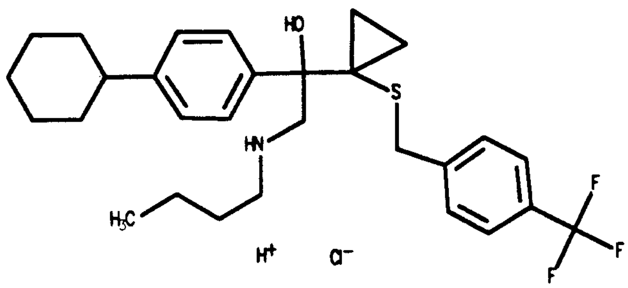

Fp.: 139°C

| ber.: | | gef.: | |
|---|---|---|---|
| C | 64.25% | C | 64.3 |
| H | 7.25% | H | 7.4 |
| Cl | 6.54% | N | 2.6 |
| F | 10.51% | | |
| N | 2.58% | | |
| O | 2.95% | | |
| S | 5.91% | | |

1.55

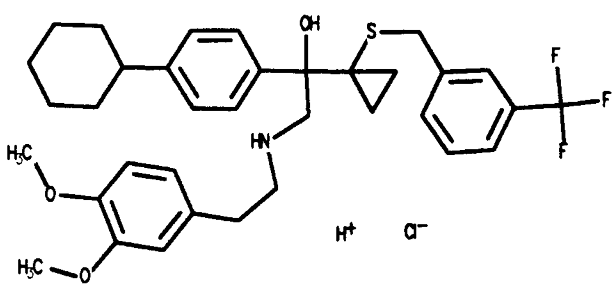

Fp.: 126°C

| ber.: | | gef.: | |
|---|---|---|---|
| C | 64.65% | C | 64.7 |
| H | 6.67% | H | 6.6 |
| Cl | 5.45% | N | 2.2 |
| F | 8.77% | | |
| N | 2.15% | | |
| O | 7.38% | | |
| S | 4.93% | | |

1.56

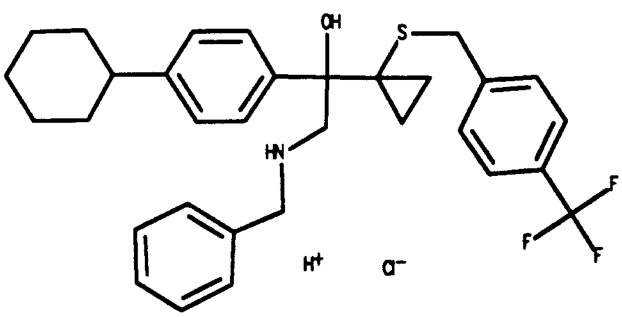

Fp.: 240°C

| ber.: | | gef.: | |
|---|---|---|---|
| C | 66.71% | C | 66.8 |
| H | 6.47% | H | 6.4 |
| Cl | 6.15% | N | 2.5 |
| F | 9.89% | | |
| N | 2.43% | | |
| O | 2.78% | | |
| S | 5.57% | | |

1.57

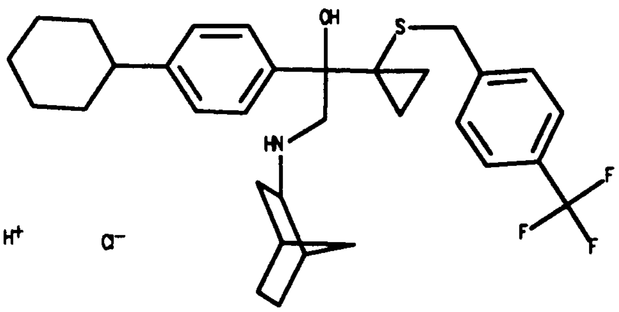

Fp.: 210°C

| ber.: | | gef.: | |
|---|---|---|---|
| C | 66.25% | C | 66.4 |
| H | 7.12% | H | 7.0 |
| Cl | 6.11% | N | 2.5 |
| F | 9.82% | | |
| N | 2.41% | | |
| O | 2.76% | | |
| S | 5.53% | | |

1.58

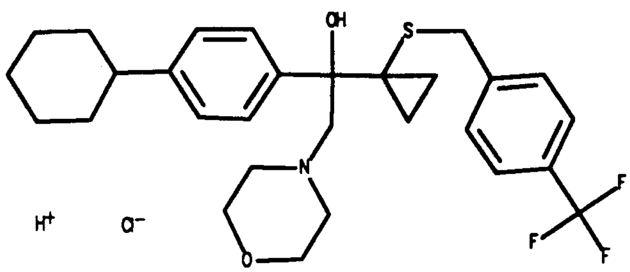

Fp.: 224°C

| ber.: | | gef.: | |
|---|---|---|---|
| C | 62.63% | C | 62.7 |
| H | 6.71% | H | 6.8 |
| Cl | 6.37% | N | 2.4 |
| F | 10.25% | | |
| N | 2.52% | | |
| O | 5.75% | | |
| S | 5.77% | | |

1.59

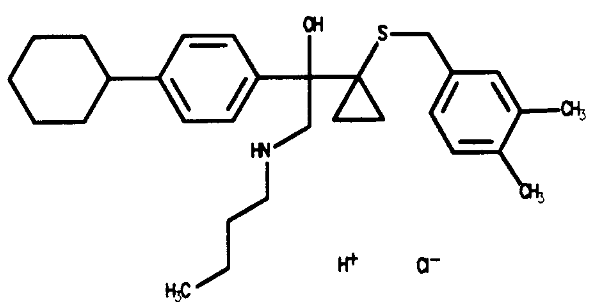

Fp.: 152°C

| ber.: | | gef.: | |
|---|---|---|---|
| C | 71.75% | C | 71.8 |
| H | 8.83% | H | 8.9 |
| Cl | 7.06% | N | 2.9 |
| N | 2.79% | | |
| O | 3.19% | | |
| S | 6.38% | | |

1.60

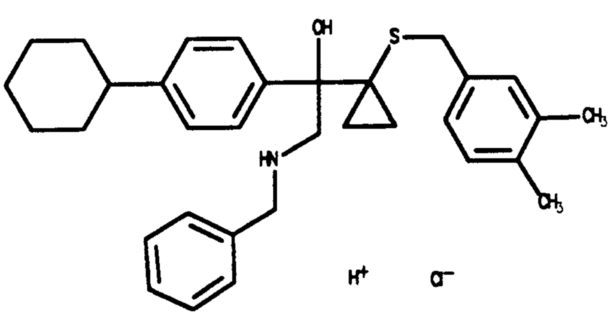

Fp.: 220°C

| ber.: | | gef.: | |
|---|---|---|---|
| C | 73.92% | C | 73.8 |
| H | 7.89% | H | 7.8 |
| Cl | 6.61% | N | 2.6 |
| N | 2.61% | | |
| O | 2.98% | | |
| S | 5.98% | | |

1.61

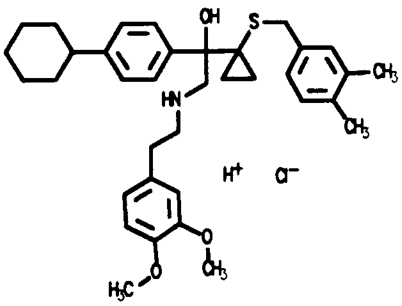

Fp.: Öl

| ber.: | | gef.: | |
|---|---|---|---|
| C | 70.85% | C | 71.0 |
| H | 7.93% | H | 7.9 |
| Cl | 5.81% | N | 2.3 |
| N | 2.30% | | |
| O | 7.86% | | |
| S | 5.25% | | |

1.62

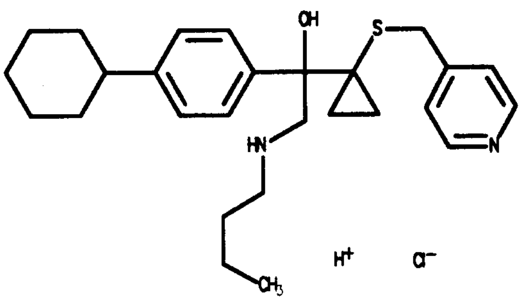

Fp.: 115°C

| ber.: | | gef.: | |
|---|---|---|---|
| C | 68.25% | C | 68.1 |
| H | 8.27% | H | 8.3 |
| Cl | 7.46% | N | 6.0 |
| N | 5.90% | | |
| O | 3.37% | | |
| S | 6.75% | | |

1.63

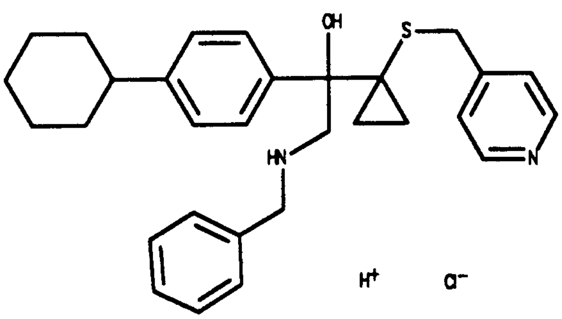

Fp.: 218°C

| ber.: | | gef.: | |
|---|---|---|---|
| C | 70.77% | C | 70.9 |
| H | 7.32% | H | 7.4 |
| Cl | 6.96% | N | 5.4 |
| N | 5.50% | | |
| O | 3.14% | | |
| S | 6.30% | | |

1.64

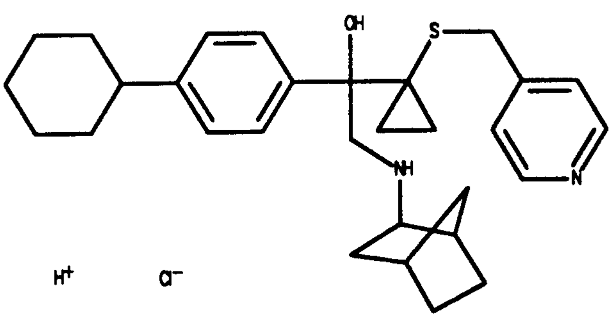

Fp.: 259°C

| ber.: | | gef.: | |
|---|---|---|---|
| C | 70.21% | C | 70.1 |
| H | 8.05% | H | 8.1 |
| Cl | 6.91% | N | 5.5 |
| N | 5.46% | | |
| O | 3.12% | | |
| S | 6.25% | | |

1.65

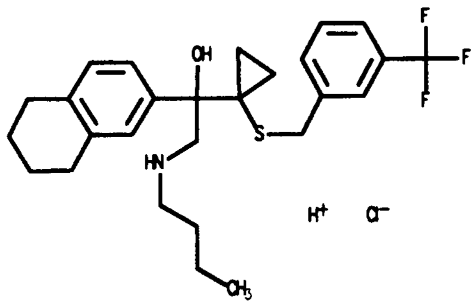

Fp.: Öl

| ber.: | | gef.: | |
|---|---|---|---|
| C | 63.08% | C | 63.0 |
| H | 6.86% | H | 6.9 |
| Cl | 6.90% | N | 2.8 |
| F | 11.09% | | |
| N | 2.72% | | |
| O | 3.11% | | |
| S | 6.24% | | |

1.66

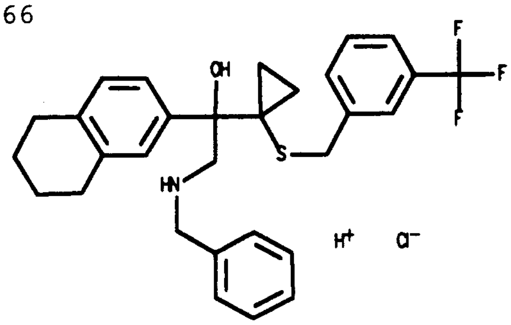

Fp.: Harz

| ber.: | | gef.: | |
|---|---|---|---|
| C | 65.74% | C | 65.8 |
| H | 6.07% | H | 6.1 |
| Cl | 6.47% | N | 2.5 |
| F | 10.40% | | |
| N | 2.56% | | |
| O | 2.92% | | |
| S | 5.85% | | |

1.67

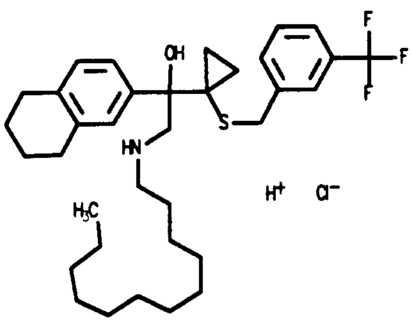

Fp.: Öl

| ber.: | | gef.: | |
|---|---|---|---|
| C | 67.12% | C | 67.0 |
| H | 8.21% | H | 8.2 |
| Cl | 5.66% | N | 2.3 |
| F | 9.10% | | |
| N | 2.24% | | |
| O | 2.55% | | |
| S | 5.12% | | |

1.68

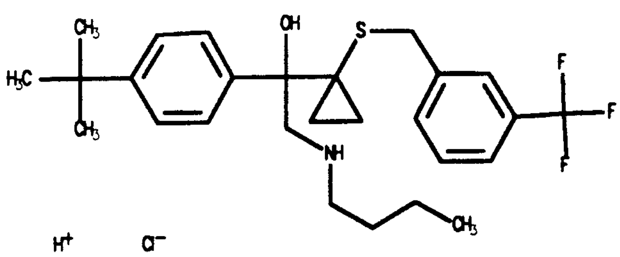

Fp.: Öl

| ber.: | | gef.: | |
|---|---|---|---|
| C | 62.84% | C | 62.6 |
| H | 7.23% | H | 7.1 |
| Cl | 6.87% | N | 2.8 |
| F | 11.04% | | |
| N | 2.71% | | |
| O | 3.10% | | |
| S | 6.21% | | |

1.69

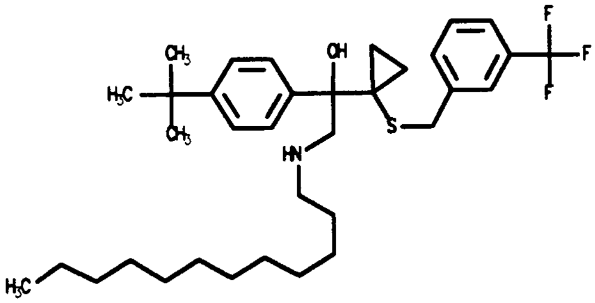

Fp.: Öl

| ber.: | | gef.: | |
|---|---|---|---|
| C | 71.03% | C | 71.0 |
| H | 8.86% | H | 8.7 |
| F | 9.63% | N | 2.2 |
| N | 2.37% | | |
| O | 2.70% | | |
| S | 5.42% | | |

1.70

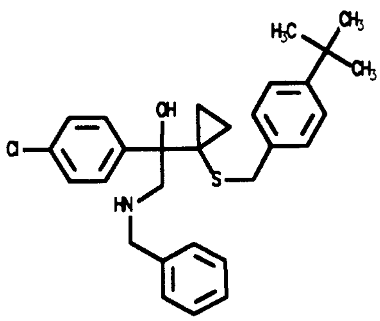

Fp.: Öl

| ber.: | | gef.: | |
|---|---|---|---|
| C | 72.55% | C | 72.4 |
| H | 7.14% | H | 7.1 |
| Cl | 7.38% | N | 2.9 |
| N | 2.92% | | |
| O | 3.33% | | |
| S | 6.68% | | |

1.71

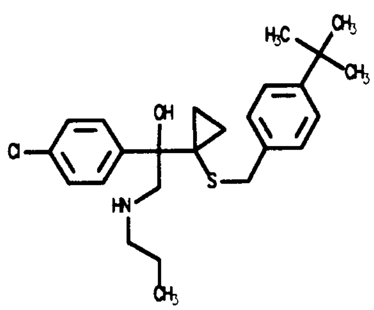

Fp.: Öl

| ber.: | | gef.: | |
|---|---|---|---|
| C | 69.50% | C | 69.5 |
| H | 7.93% | H | 8.0 |
| Cl | 8.21% | N | |
| N | 3.24% | | |
| O | 3.70% | | |
| S | 7.42% | | |

1.72

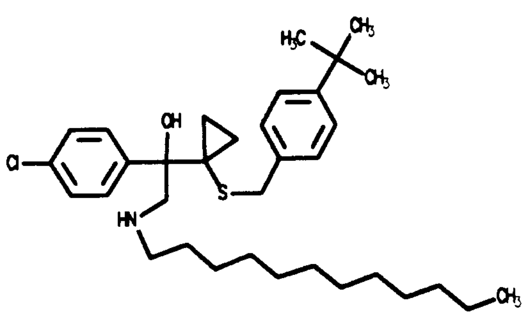

Fp.: Öl

| ber.: | | gef.: | |
|---|---|---|---|
| C | 73.14% | | |
| H | 9.39% | | |
| Cl | 6.35% | | |
| N | 2.51% | | |
| O | 2.87% | | |
| S | 5.74% | | |

1.73

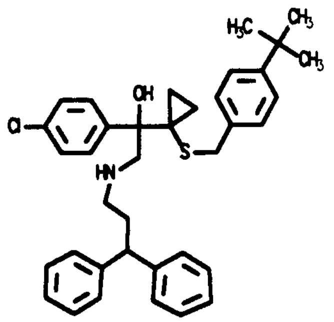

Fp.: Öl

| ber.: | | gef.: | |
|---|---|---|---|
| C | 76.06% | C | 76.0 |
| H | 7.25% | H | 7.3 |
| Cl | 6.07% | N | 2.3 |
| N | 2.40% | | |
| O | 2.74% | | |
| S | 5.49% | | |

1.74

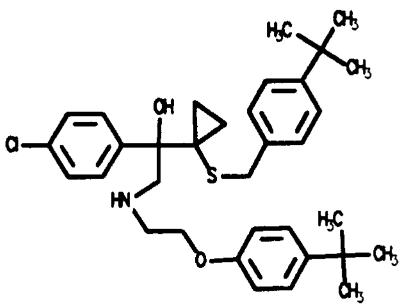

Fp.: Öl

| ber.: | | gef.: | |
|---|---|---|---|
| C | 72.12% | C | 72.0 |
| H | 7.83% | H | 7.9 |
| Cl | 6.26% | N | 2.5 |
| N | 2.47% | | |
| O | 5.65% | | |
| S | 5.66% | | |

1.75

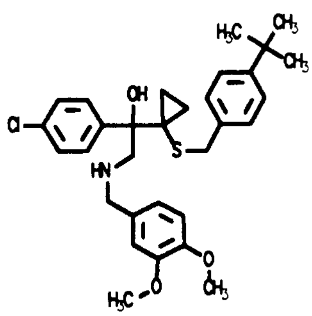

Fp.: Öl

| ber.: | | gef.: | |
|---|---|---|---|
| C | 68.93% | C | 68.9 |
| H | 7.09% | H | 7.1 |
| Cl | 6.56% | N | 2.6 |
| N | 2.59% | | |
| O | 8.89% | | |
| S | 5.94% | | |

1.76

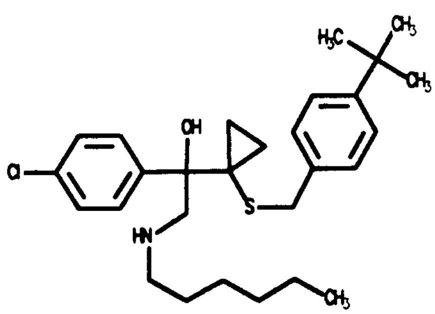

Fp.: Öl

| ber.: | | gef.: | |
|---|---|---|---|
| C | 70.93% | C | 70.0 |
| H | 8.50% | H | 8.3 |
| Cl | 7.48% | N | 2.9 |
| N | 2.95% | | |
| O | 3.37% | | |
| S | 6.76% | | |

1.77

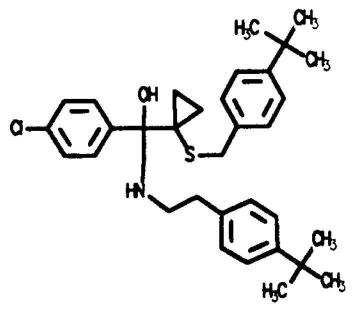

Fp.: Öl

| ber.: | | gef.: | |
|---|---|---|---|
| C | 74.22% | C | 74.2 |
| H | 8.06% | H | 8.1 |
| Cl | 6.44% | N | 2.4 |
| N | 2.55% | | |
| O | 2.91% | | |
| S | 5.83% | | |

1.78

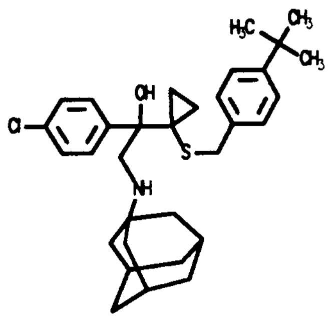

Fp.: Öl

| ber.: | | gef.: | |
|---|---|---|---|
| C | 73.32% | C | 73.1 |
| H | 8.08% | H | 8.0 |
| Cl | 6.76% | N | 2.7 |
| N | 2.67% | | |
| O | 3.05% | | |
| S | 6.12% | | |

1.79

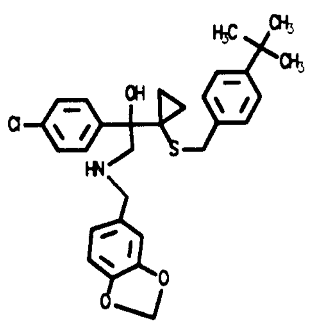

Fp.: Öl

| ber.: | | gef.: | |
|---|---|---|---|
| C | 68.75% | C | 68.7 |
| H | 6.54% | H | 6.6 |
| Cl | 6.76% | N | 2.7 |
| N | 2.67% | | |
| O | 9.16% | | |
| S | 6.12% | | |

1.80

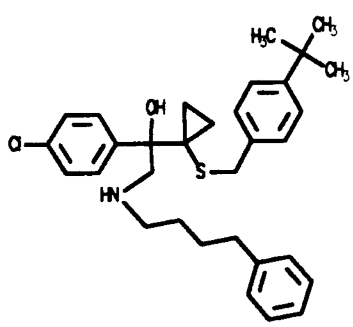

Fp.: Öl

| ber.: | | gef.: | |
|---|---|---|---|
| C | 73.60% | C | 73.7 |
| H | 7.72% | H | 7.8 |
| Cl | 6.79% | N | 2.8 |
| N | 2.68% | | |
| O | 3.06% | | |
| S | 6.14% | | |

1.81

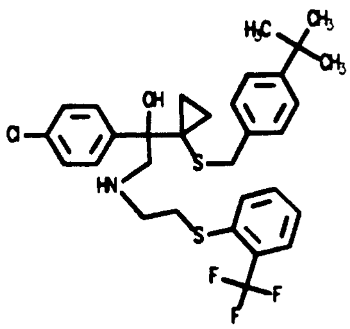

Fp.: Öl

| ber.: | | gef.: | |
|---|---|---|---|
| C | 62.66% | C | 62.7 |
| H | 5.94% | H | 6.0 |
| Cl | 5.97% | N | 2.3 |
| F | 9.59% | | |
| N | 2.36% | | |
| O | 2.69% | | |
| S | 10.79% | | |

1.82

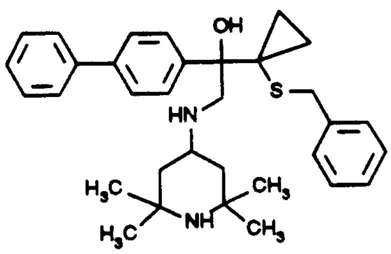

| ber.: | | gef.: | |
|---|---|---|---|
| C | 76.99 | C | 76.2 |
| H | 8.22 | H | 8.1 |
| N | 5.44 | N | 5.4 |
| O | 3.11 | | |
| S | 6.23 | | |

1.83

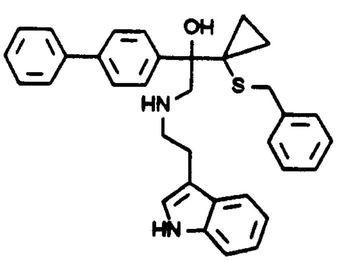

| ber.: | | gef.: | |
|---|---|---|---|
| C | 78.73 | C | 78.6 |
| H | 6.61 | H | 6.5 |
| N | 5.40 | N | 5.4 |
| O | 3.08 | | |
| S | 6.18 | | |

1.84

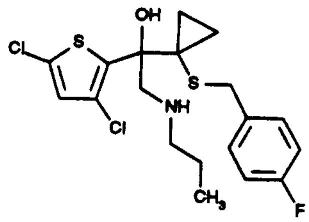

Fp.: Öl

| ber.: | | gef.: | |
|---|---|---|---|
| C | 52.53 | C | 52.4 |
| H | 5.10 | H | 5.0 |
| N | 3.22 | N | 3.3 |
| Cl | 16.32 | | |
| O | 3.68 | | |
| F | 4.37 | | |
| S | 14.76 | | |

1.85

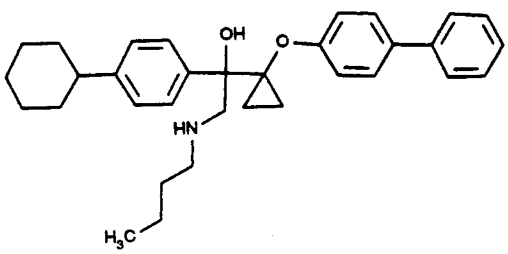

Fp.: Öl

| ber.: | | gef.: | |
|---|---|---|---|
| C | 81.95 | C | 88.1 |
| H | 8.54 | H | 8.5 |
| N | 2.90 | N | 3.0 |
| O | 6.61 | | |

1.86

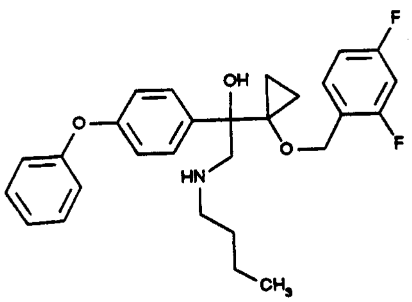

Fp.: Öl

| ber.: | | gef.: | |
|---|---|---|---|
| C | 71.93 | C | 72.1 |
| H | 6.68 | H | 6.6 |
| N | 2.99 | N | 3.1 |
| O | 10.27 | | |

1.87

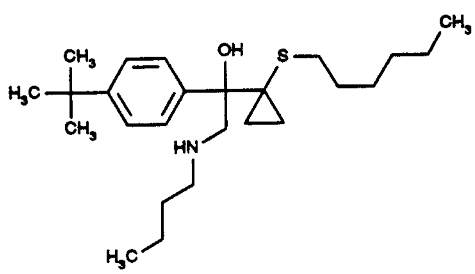

Fp.: Harz

| ber.: | | gef.: | |
|---|---|---|---|
| C | 74.02 | C | 74.2 |
| H | 10.68 | H | 10.6 |
| N | 3.45 | N | 3.6 |
| O | 3.94 | | |
| S | 7.90 | | |

1.88

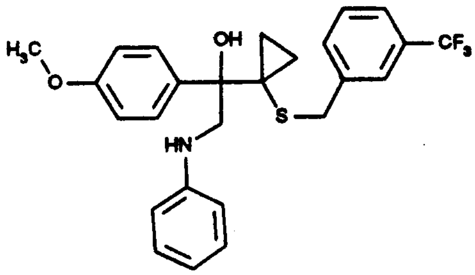

Fp.: Öl

| ber.: | | gef.: | |
|---|---|---|---|
| C | 65.94 | C | 65.8 |
| H | 5.53 | H | 5.5 |
| N | 2.96 | N | 2.9 |
| O | 6.76 | | |
| S | 6.77 | | |
| F | 12.04 | | |

1.89

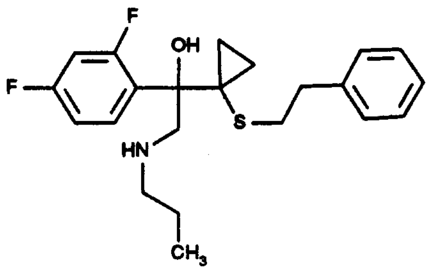

Fp.: Öl

| ber.: | | gef.: | |
|---|---|---|---|
| C | 67.49 | C | 67.6 |
| H | 6.95 | H | 7.0 |
| N | 3.58 | N | 3.5 |
| F | 9.70 | | |
| O | 4.09 | | |
| S | 8.19 | | |

1.90

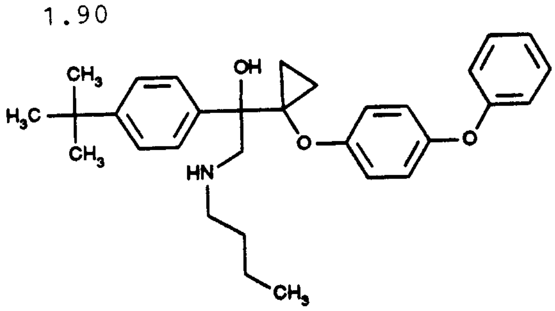

Fp.: Öl

| ber.: | | gef.: | |
|---|---|---|---|
| C | 78.61 | C | 78.5 |
| H | 8.30 | H | 8.4 |
| N | 2.96 | N | 3.1 |
| O | 10.13 | | |

1.91

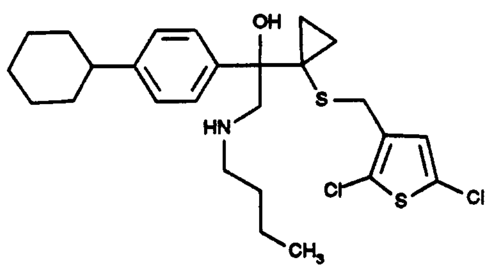

Fp.: Harz

| ber.: | | gef.: | |
|---|---|---|---|
| C | 60.92 | C | 61.1 |
| H | 6.88 | H | 6.9 |
| N | 2.73 | N | 2.7 |
| O | 3.12 | | |
| Cl | 13.83 | | |
| S | 12.51 | | |

1.92

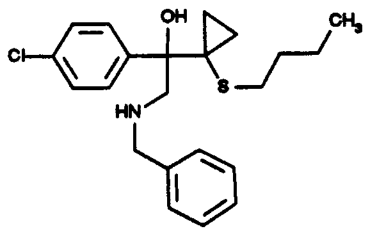

Fp.: Öl

| ber.: | | gef.: | |
|---|---|---|---|
| C | 67.77 | C | 67.6 |
| H | 7.24 | H | 7.2 |
| N | 3.59 | N | 3.7 |
| Cl | 9.09 | | |
| O | 4.10 | | |
| S | 8.22 | | |

1.93

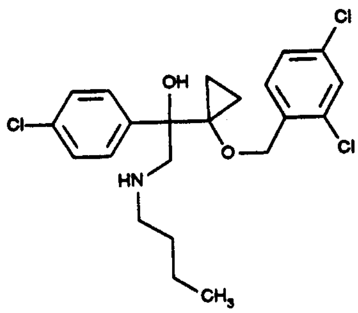

Fp.: Harz

| ber.: | | gef.: | |
|---|---|---|---|
| C | 59.67 | C | 59.8 |
| H | 5.92 | H | 6.0 |
| N | 3.16 | N | 3.2 |
| Cl | 24.02 | | |
| O | 7.23 | | |

1.94

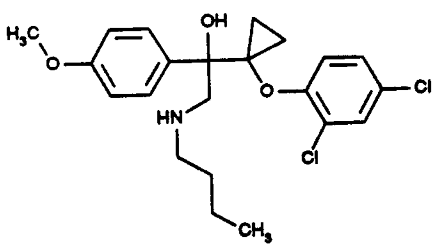

Fp.: Öl

| ber.: | | gef.: | |
|---|---|---|---|
| C | 62.27 | C | 62.4 |
| H | 6.41 | H | 6.5 |
| N | 3.30 | N | 3.3 |
| O | 11.31 | | |
| Cl | 16.71 | | |

1.95

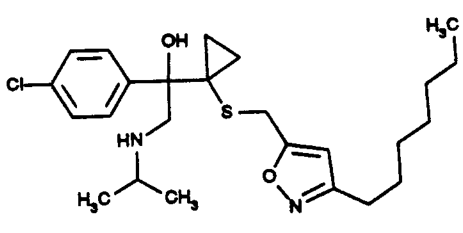

Fp.: Öl

| ber.: | | gef.: | |
|---|---|---|---|
| C | 64.56 | C | 64.5 |
| H | 8.02 | H | 8.2 |
| N | 6.02 | N | 6.0 |
| Cl | 7.62 | | |
| O | 6.88 | | |
| S | 6.89 | | |

Die Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Hierbei wird von der Hefe Candida albicans insbesondere das Exoenzymsystem dergestalt beeinflußt, daß die Pathogenität der Erreger deutlich absinkt. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:
Dermatomykosen und Systemmykosen durch Trichophyton mentagrophytes und andere Trichophytonarten, Mikrosporenarten, Epidermophyton floccosum, und biphasische Pilze sowie Schimmelpilze hervorgerufen. Insbesondere werden tiefe Mykosen, die durch Candida albicans hervorgerufen werden, günstig beeinflußt, da hierbei ein Eindringen der Pilze in die Wirtszelle verhindert bzw. erschwert wird.

Als Indikationsgebiete in der Tiermedizin können beispielsweise aufgeführt werden:
Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere Wirkstoffe enthalten oder die aus einem oder mehreren erfindungsgemäß verwendeten Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Ein Hemmstoff für die unterschiedlichen Phospholipasen von Candida albicans muß im Patienten überall dort in hinreichenden Konzentrationen vorliegen, wo der Pilz die Parenchyme besiedeln kann. Dieser Umstand setzt voraus, daß die entsprechenden Substanzen in einer Konzentration verabreicht werden müssen, die sich zuvor in Tierexperimenten als wirksam erwiesen hat.

Bei den schweren Krankheitsbildern der tiefen Candidose befinden sich die Patienten meist in einem sehr schlechten Allgemeinzustand. Hohes Fieber und weitere Erkrankungen sind häufig anzutreffen. Bei den Dosierungsvorgaben muß zwischen der prophylaktischen Gabe und der Therapie im nachgewiesenen Infektionsfall unterschieden werden. Bei der Prophylaxe kann von einem besseren Allgemeinzustand der Patienten ausgegangen werden, der eine orale Verabreichung ermöglicht. Hierbei können Tabletten, Lösungen, Gele oder Trockensaft zum Einsatz kommen. Bei den Formen mit nachgewiesenen tiefen Candidosen muß oft davon ausgegangen werden, daß eine geregelte orale Aufnahme der Wirkstoffe nicht immer gewährleistet ist. Hierfür kommen dann parenterale Anwendungsformen in Frage. Im Ausnahmefall kann auch an eine subcutane Verabreichung gedacht werden.

Als Kandidaten für eine Prophylaxe kommen in erster Linie immunkomprimierte Patienten in Frage, die durch entsprechende medikamentöse Belastung oder durch körpereigene Immunprobleme in dieser Situation sind. Dies sind insbesondere Transplantationspatienten, Zuckerkranke und/oder adipöse Patienten, AIDS-Patienten, unter Chemotherapie stehende Patienten, Langzeitbeatmete usw..

Die Verbindungen zeigen Hemmungen der Phospholipase von Candida albicans, die weit unter der in vitro festgestellten minimalen inhibitorischen Konzentrationen der Wirkstoffe gegenüber Candida albicans liegen. Daher kann die Dosierung im Regelfall unter derjenigen liegen, die für eine reine antimykotische Therapie nötig wäre.

Die Wirkung im Patienten beruht darauf, daß die Wirkstoffe bei den Candida Zellen, die sich in der Nähe der Parenchyme befinden, zwei unterschiedliche Effekte auslöst. Zum einen wird die Adhäsion der Hefezellen an die Körperzellen verhindert und zum anderen wird Candida albicans daran gehindert, die Körperzellen mit Keimschläuchen zu penetrieren. Durch dieses duale Wirkungskonzept kann die Hefe ihre Pathogenität nicht zur vollen Ausprägung bringen. Allerdings muß erwähnt werden, das Candida albicans außer den Phospholipasen noch weitere Pathomechanismen wie z.B. die Protease besitzt. Die Anheftung an körpereigene Zellen ist jedoch der primäre Schritt zur Penetration. Da diese Anheftung durch Phospholipasehemmer verhindert wird, können die anderen Pathomechanismen nicht voll zur Geltung kommen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die prophylaktisch und therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäß verwendeten Wirkstoffen auch weitere pharmazeutische Wirkstoffe enthalten.

Die Herstellung der oben aufgeführten pharmazeutischen Zubereitungen erfolgt in üblicher Weise nach

bekannten Methoden, z.B. durch Mischen des Wirkstoffs oder der Wirkstoffe mit dem Trägerstoff oder den Trägerstoffen.

Zur vorliegenden Erfindung gehört die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäß verwendeten Wirkstoffe in Gesamtmengen von mindestens etwa 0,05, vorzugsweise 0,1 , insbesondere 0,5 mg/kg Körpergewicht bis höchstens etwa 200, vorzugsweise bis 100, insbesondere bis 10 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen, bei tiefen Mykosen jedoch über wesentlich längere Zeiträume (bis zu 6 Wochen) verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die Verbindungen der Formel I sind auch als Biozide wirksam. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der Verbindungen I erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und echte Mehltaupilze im Obst-, Gemüse-, Getreide- und Zierpflanzenbau.

Die Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinapthylmethan-6,6'-di-sulfonsaures Natrium, dibutylnaphthalinsulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:
alkylarylsulfonsaure Calciumsalze, wie Ca-dodecylbenzolsulfonat, oder nicht ionische Emulgatoren wie Fettsäurepolyglykolester, Alkylarylpolyglykolether, Fettalkoholpolyglykolether, Propylenoxid- Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinit oder von granuliertem Inertmaterial. Auch können geeignete Wirkstoffe in der für die Herstellung von Düngemittelgranulaten üblichen Weise - gewünschtenfalls in Mischung mit Düngemitteln, granuliert werden.

In Spritzpulvern beträgt die Wirkstoffkonzentration z.B. etwa 10-90 Gew.-%, der Rest zu 100 Gew.-%

besteht aus üblichen Formulierungsbestandteilen. Bei emulgierbaren Konzentraten kann die Wirkstoffkonzentration etwa 10-80 Gew.-% an Wirkstoff, bei versprühbaren Lösungen etwa 1-20 Gew.-% betragen. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z.B. Insektiziden, Akariziden, Herbiziden, Düngemitteln, Wachstumsregulatoren oder weiteren Fungiziden sind gegebenenfalls möglich, wobei u.U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispergierbares, benetzbares Pulver wird erhalten, indem man 25 Gewichtsteile Wirkstoff, 65 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377 ° C) mischt und in einer Reibkugelmühle auf eine Feinheit von unter 5 Mikron vermahlt.

Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertem Nonylphenol (10 Ae0) als Emulgator.

Als Beispiel für die Hemmung der pathogenen Phase dimorpher Hefezellen werden Ergebnisse eines in-vitro-Enzymtests angeführt, in welchen die prozentuale Hemmung freigesetzter Exoenzyme, insbesondere freigesetzter Lysophospholipase (Phospholipase B), als Maß für die Wirksamkeit bestimmt wird.

Zur Feststellung der Enzymhemmung wurde eine Suspension von Candida albicans Blastokonidien (Stamm 200/175), wobei die Keimdichte photometrisch auf eine Extinktion von 0,5 (500 nm) eingestellt war, mit Präparatelösung bzw. zur Kontrolle mit Lösungsmittellösung vermischt und zwar

a) 100 µl Präparatelösung (Suspension)
+ 900 µl Keimsuspension

b) 100 µl Lösungsmittel
+ 900 µl Keimsuspension

Jeweils 5 µl der 30 min bei 21 ° C inkubierten Blastokonidiensuspension wurden auf eine Agarplatte (Sabourand Agar mit Zusatz von 8 % Eigelb, 1 M NaCl, 5 mM $CaCl_2$) aufgetropft.

Die so beimpfte Platte wurde 3 Tage bei 37 ° C bebrütet.

Die Auswertung erfolgte derart, daß

1. der Durchmesser (mm) der Candida albicans-Kolonie (behandelt und unbehandelt) sowie
2. der Gesamtdurchmesser von Kolonie und Trübungshof, welcher durch Exoenzyme verursacht wurde (behandelt und unbehandelt) bestimmt wurde.

Aus der Bestimmung des Quotienten von Präparate- und Kontrollgruppe ergab sich ein Wert, der als Maß für die Enzymaktivität anzusehen war.

Wie aus Tabelle 2 ersichtlich, hemmen die erfindungsgemäßen Verbindungen die freigesetzten Exoenzyme wesentlich stärker als Propranolol.

Propanolol wird in der Literatur (Pappu A.S. et al. in Biochem. Pharmacol. 34, 521-24, 1985) als wirksamste Substanz beschrieben, die gegenüber Phospholipase aus Leberzellen in einem entsprechenden in-vitro-Test Hemmwirkung zeigte.

Der Stand der Technik wird durch die erfindungsgemäßen Verbindungen I überraschenderweise deutlich übertroffen.

Tabelle 2 Phospholipase-Hemmung einiger Verbindungen der Formel I

| Verbindung | Konzentration (Mikrogramm/ml) | % Hemmung |
|---|---|---|
| 1.4 | 100 | 100 |
| | 50 | 100 |
| | 10 | 20 |
| 1.5 | 100 | 100 |
| | 50 | 100 |
| | 10 | 50 |
| 1.15 | 100 | 100 |
| | 50 | 100 |
| | 10 | 25 |
| 1.24 | 100 | 100 |
| | 50 | 100 |
| | 10 | 75 |
| | 5 | 25 |
| | 1 | 40 |
| 1.25 | 100 | 100 |
| | 50 | 100 |
| | 10 | 68 |
| | 5 | 25 |
| | 1 | 22 |
| 1.26 | 100 | 100 |
| | 50 | 100 |
| | 10 | 85 |
| | 5 | 50 |
| 1.27 | 100 | 100 |
| | 50 | 100 |
| | 10 | 87 |
| | 5 | 62 |
| | 1 | 11 |
| 1.29 | 100 | 100 |
| | 50 | 100 |
| | 10 | 67 |
| | 5 | 50 |

Fortsetzung Tabelle 2

| Verbindung | Konzentration (Mikrogramm/ml) | % Hemmung |
|---|---|---|
| 1.34 | 100 | 100 |
| | 50 | 100 |
| | 10 | 100 |
| | 5 | 67 |
| Propranolol | 100 | 30 |

Als Beispiele für die systemische in vivo-Wirksamkeit der Verbindungen dient die systemische Candida albicans Infektion bei der Maus.

Ziel dieser Methode ist es, den Effekt von Präparaten auf eine systemische Candida albicans Infektion zu ermitteln. Die Infektion führt innerhalb von 2-4 Tagen zum Tod der Tiere.

Beschreibung der Methode: Albino Mäuse (NMRI, Männchen, 15-20 g Körpergewicht) werden intravenös mit Candida albicans Hefezellen infiziert (Stamm 200/175), die frisch auf Malzagar vorkultiviert wurden und in physiologischer Kochsalzlösung suspendiert wurden. Die Infektionsdosis, die in dem angegebenen Zeitrahmen zur Lethalität führt, wurde photometrisch eingestellt und enthielt eine Million Hefezellen pro Maus.

Die zu prüfenden Verbindungen werden entweder alleine oder in Kombination mit einem Standardantimykotikum verabreicht. Im durchgeführten Test wurde ein Kombinationspräparat verwendet, wobei als Standardantimykotikum Fluconazol (Fa. Pfizer) diente. Applikationswege sind je nach Substanzgruppe unterschiedlich, wobei der oralen Applikation der Vorzug gegeben wird. Die Mäuse werden 4 Wochen beobachtet, die Mortalität wird täglich registriert, und die Überlebenszeiten der einzelnen Gruppen werden gemittelt und untereinander verglichen. Gruppengröße beträgt durchschnittlich 10 Tiere.

Der Vergleich der Mittelwerte geschieht mit dem Students T Test.

Wie Tabelle 3 zeigt, verlängert sich bei Kombinationstherapie mit Verbindung 1.4 (14 x 50 mg/kg p.o. 1 x täglich) die Überlebenszeit von mit candida infizierten Mäusen um 57 %. (Die Überlebenszeit der mittels Fluconazol-Monotherapie behandelten Tiere wurde als 100 definiert, worauf sich für die Kombinationstherapie 157 ergaben).

Tabelle 3 Überlebenszeiten von mit Candida infizierten Mäusen bei Kombinationstherapie mit Fluconazol

| Verbindung | Überlebenszeit (bez. auf Fluconazol = 100 %) |
|---|---|
| Fluconazol | 100 % |
| Fluconazol + Verbindung 1.4 | 157 % |

Dosierungen:
Fluconazol jeweils 9 x p.o. 50 mg/kg/Tag Kombinationspartner jeweils 14 x p.o. 50 mg/kg /Tag

Die Fungizide (pilzabtötende Wirkung) der Verbindungendes Typs der Formel I wird bestimmt, indem Ruhestadien (Vermehrungsform ist gleich Mikrokonidien) des Pilzes Trichophyton Mentagrophytes in aqua dest. über Nacht inkubiert werden. Nach Auswaschen des Präparats (vgl. H. Hänel und W. Raether in mycoses 31 (3), 148-154 (1988)) wird auf Überleben der Pilze geprüft.

Einige auf diese Weise bestimmte Werte sind in Tabelle 4 wiedergegeben (Vergleichspräparat ist Rilopirox).

Die erfindungsgemäßen Verbindungen liegen damit bezüglich ihrer Fungizidie im Bereich des hochaktiven Rilopirox.

Tabelle 4

| Verbindung | µg/ml | | | | |
|---|---|---|---|---|---|
| | 80 | 40 | 20 | 10 | 5 |
| 1.17 | 100 | 100 | 100 | 96.9 | |
| 1.29 | 100 | 100 | 100 | 99.66 | |
| 1.35 | 100 | 100 | 100 | 100 | 99.29 |
| 1.36 | 100 | 100 | 100 | 96.47 | |
| 1.44 | 100 | 100 | 100 | 99.09 | |
| 1.48 | 100 | 100 | 100 | 99.44 | |
| Rilopirox | 100 | 100 | 100 | 100 | 96.2 |

## Patentansprüche

**1.** Verbindung der Formel I

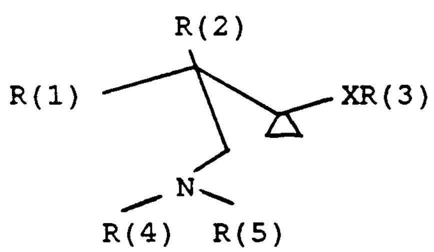

I

in welcher bedeuten:

R(1) t-Butyl, Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche

F, Cl, Br, J, ($C_1$-$C_{18}$)-Alkyl (geradkettig oder verzweigt und unsubstituiert oder mit 1-9 F-, Cl-Atomen substituiert), ($C_3$-$C_{18}$)-Cycloalkyl [mono-, bi- oder multicyclisch, unsubstituiert oder ein- oder zweifach substituiert mit ($C_1$-$C_4$)-Alkyl (geradkettig oder verzweigt), ($C_1$-$C_4$)-Alkoxy (geradkettig oder verzweigt), $CF_3$, F, Cl, Br, OH Y-($C_1$-$C_{18}$)-Alkyl (geradkettig oder verzweigt), Y-($C_3$-$C_{18}$)-Cycloalkyl [mono-, bi- oder multicyclisch, unsubstituiert oder wie oben angegeben substituiert], ($C_2$-$C_{15}$)-Alkenyl (geradkettig oder verzweigt, ein- oder mehrfach ungesättigt), Y-($C_2$-$C_{15}$)-Alkenyl (geradkettig oder verzweigt, ein- oder mehrfach ungesättigt), Y-Phenyl, Y-($C_1$-$C_2$)-Alkyl-phenyl, Phenyl-($C_1$-$C_2$)-alkyl, Biphenylyl, CN, $NO_2$, $CO_2Z$ (mit Z gleich Phenyl, ($C_1$-$C_{15}$)-Alkyl, ($C_2$-$C_{15}$)-Alkenyl, wobei die aufgeführten Alkyl- und Alkenylsubstituenten geradkettig oder verzweigt sind und Phenyl unsubstituiert ist oder 1-3 Substituenten aus der Reihe F, Cl, $CF_3$, OH trägt), bedeuten,

mit Y gleich Sauerstoff, Schwefel, Sulfinyl, Sulfonyl,

R(2) OH, F, Cl, Br, ($C_1$-$C_{10}$)-Alkylcarbonyloxy (geradkettig oder verzweigt), ($C_1$-$C_{10}$)-Alkyloxy (geradkettig oder verzweigt), Benzyloxy (unsubstituiert oder 1-2fach substituiert mit F, Cl, Br, $CF_3$, $OCH_3$), Phenylcarbonyloxy, wobei der Phenylrest unsubstituiert ist oder mit 1-3 Substituenten aus der Reihe F, Cl, Br, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1-9fach substituiert durch F, Cl, Br, J und/oder $OCH_3$), ($C_2$-$C_{12}$)-Alkenyl (geradkettig oder verzweigt, ein- oder mehrfach ungesättigt, in Form des reinen E- oder Z-Diastereomeren oder als E/Z-Diastereomerengemisch, unsubstituiert oder 1- bis 9-fach substituiert durch F, Cl, Br, J und/oder $OCH_3$), ($C_2$-$C_{12}$)-Alkinyl (geradkettig oder verzweigt, mit einer oder mehreren Dreifachbindungen, unsubstituiert oder 1- bis 9-fach substituiert durch F, Cl, Br, J und/oder $OCH_3$), ($C_3$-$C_{12}$)-Alkeninyl (geradkettig oder verzweigt, eine oder mehrere Doppel- und/oder Dreifachbindungen enthaltend, in Form des reinen E- oder Z-Diastereomeren oder als E/Z-Diastereomerengemisch, unsubstituiert oder 1- bis 9-fach substituiert durch F, Cl, Br, J und/oder $OCH_3$), ($C_3$-$C_{12}$)-Cycloalkyl (unsubstituiert oder 1- bis 9-fach substituiert durch F, Cl, Br, J, $OCH_3$ und/oder $CH_3$), ($C_3$-$C_{12}$)-Cycloalkyl-($C_1$-$C_3$)-alkyl (unsubstituiert oder 1-9-fach substituiert durch F, Cl, Br und/oder $CH_3$), Phenyl, Benzyl, Phenyl-($C_2$-$C_4$)-alkyl, Phenoxyphenyl, Phenylthiophenyl, Phenylsulfinylphenyl, Phenylsulfonylphenyl, Phenoxyphenyl-($C_1$-$C_4$)-alkyl, Phenylthiophenyl-($C_1$-$C_4$)-alkyl, Naphthyl, Naphthyl-($C_1$-$C_4$)-alkyl, Biphenylyl, Biphenyl-($C_1$-$C_4$)-alkyl, Hetaryl, Hetaryl-($C_1$-$C_2$)-alkyl (mit Hetaryl gleich Thiophen, Furan, Pyridin, Oxazol, Isoxazol, Thiazol, Isthiazol, Pyrrol, 1,2,4-Oxadiazol, 1,3,4-Oxadiazol, Pyrimidin, Pyrazin, Benzothiofuran, Benzothiazol, Benzoxazol, Indol, Chinolin und Isochinolin), wobei die genannten aromatischen Ringsysteme und Hetarylsysteme unsubstituiert oder 1- bis 3-fach substituiert sind durch F, Cl, Br, $CF_3$, ($C_1$-$C_{10}$)-Alkyl (geradkettig oder verzweigt, unsubstituiert oder 1- bis 3-fach substituiert durch F, Cl und/oder $OCH_3$), ($C_3$-$C_6$)-Cycloalkyl (unsubstituiert), oder die aromatischen Ringsysteme oder die Hetarylsysteme substituiert sind durch Phenyl (unsubstituiert oder 1- bis 3-fach substituiert durch F, Cl, $OCH_3$), CN, OH, $NH_2$, NR(6)R(7), $NO_2$, Z'R(6), worin R(6) ($C_1$-$C_{10}$)-Alkyl (geradkettig oder verzweigt, unsubstituiert oder 1-bis 9-fach substituiert durch F, Cl und/oder $OCH_3$) und Z' gleich Sauerstoff oder Schwefel ist, oder R(6) ($C_3$-$C_{12}$)-Alkenyl (geradkettig oder verzweigt, ein- oder mehrfach ungesättigt, unsubstituiert oder 1- bis 3-fach substituiert durch F, Cl und/oder $OCH_3$) ist, oder R(6) Benzyl, Biphenylylmethyl, Naphthylmethyl, Phenyl, Thienylmethyl, Thienyl, ($C_1$-$C_{10}$)-Alkylcarbonyl (geradkettig oder verzweigt) oder Phenyl-carbonyl ist, wobei die hier für R(6) genannten Ringsysteme ihrerseits unsubstituiert oder 1- bis 3-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R(6) und R(7) gleich oder verschieden sein können,

R(4) H, ($C_1$-$C_{18}$)-Alkyl (geradkettig oder verzweigt), ($C_2$-$C_{10}$)-Alkenyl (geradkettig oder verzweigt, ein- oder mehrfach ungesättigt), Benzyl (unsubstituiert oder ein- oder mehrfach substituiert durch F, Cl, Br, $CF_3$, ($C_1$-$C_4$)-Alkyl (geradkettig oder verzweigt), $OCH_3$, O-Phenyl oder Phenyl),

R(5) H, ($C_1$-$C_{18}$)-Alkyl (geradkettig oder verzweigt), ($C_2$-$C_{18}$)-Alkenyl (geradkettig oder verzweigt, ein- oder mehrfach ungesättigt), ($C_3$-$C_{12}$)-Cycloalkyl, (mono-, bi- oder tricyclisch), ($C_1$-$C_8$)-Alkyl-oxy-($C_2$-$C_{10}$)-alkyl, Phenyl-($C_1$-$C_6$)-Alkyl (geradkettig oder verzweigt), Phenyl-oxy-($C_1$-$C_6$)alkyl, Phenyl-thio-($C_1$-$C_6$)-alkyl, Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Thienyl, Thienyl-methyl, Phenyl, wobei die bezüglich R(5) genannten Phenyl- oder Thienylsysteme unsubstituiert oder ein- bis 3-fach substituiert sind durch Substituenten aus der Gruppe F, Cl, Br, ($C_1$-$C_{10}$)-Alkyl (geradkettig oder verzweigt), ($C_3$-$C_8$)-Cycloalkyl, OH, SH, ($C_1$-$C_{10}$)-Alkoxy (geradkettig oder verzweigt), Phenyl, Benzyl, Phenethyl, Thiophenyl, $C_aF_{a+1}$ mit a = 1-6), -O $(CH_2)_{1-2}$O-,

oder

R(5) ist ein Piperidin-4-yl-Rest, der unsubstituiert ist oder durch 1 bis 4 Methylgruppen substituiert ist,

oder

R(5) ist ein Indol-3-yl-($C_1$-$C_4$)-Alkylrest (geradkettig oder verzweigt)

R(4) mit R(5) eine Kette aus $(CH_2)_m$-Einheiten mit m = 4-6 bilden, die durch O, S oder N-Atom

unterbrochen sein kann, wobei N als weiteren Bindungspartner ein H-Atom, eine $CH_3$, Phenyl-, Benzyl oder Phenethylgruppe trägt und der so gebildete Heterocyclus an den $CH_2$-Einheiten unsubstituiert ist oder 1- bis 4 $CH_3$-Gruppen als Substituenten trägt) und

X Sauerstoff, Schwefel, Sulfinyl oder Sulfonyl,

und ihre Salze mit pharmazeutisch akzeptablen Säuren und in Form von physiologisch hydrolysierbaren und pharmazeutisch akzeptablen Derivaten.

**2.** Verbindung I nach Anspruch 1, in der bedeuten:

R(1) Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Pyridyl,

wobei die genannten Ringsysteme unsubstituiert oder mit 1-3 Substituenten, die gleich oder verschieden sind, substituiert sind, welche

F, Cl, ($C_1$-$C_{12}$)-Alkyl (geradkettig oder verzweigt und unsubstituiert oder mit 1-6 F-, Cl-Atomen substituiert), ($C_3$-$C_{18}$)-Cycloalkyl [mono-, bi- oder multicyclisch, unsubstituiert oder einfach substituiert mit ($C_1$-$C_4$)-Alkyl (geradkettig oder verzweigt), ($C_1$-$C_4$)-Alkoxy (geradkettig oder verzweigt), $CF_3$, F, Cl, OH], Y-($C_1$-$C_{10}$)-Alkyl (geradkettig oder verzweigt), Y-($C_3$-$C_{10}$)-Cycloalkyl [mono-, bi- oder multicyclisch, unsubstituiert oder wie oben angegeben substituiert], ($C_2$-$C_{10}$)-Alkenyl (geradkettig oder verzweigt, ein- oder mehrfach ungesättigt), Y-($C_2$-$C_{10}$)-Alkenyl (geradkettig oder verzweigt, ein- oder mehrfach ungesättigt), Y-Phenyl, Y-($C_1$-$C_2$)-Alkyl-phenyl, Phenyl-($C_1$-$C_2$)-alkyl, Biphenylyl, wobei die aufgeführten Alkyl- und Alkenylsubstituenten geradkettig oder verzweigt sind und Phenyl unsubstituiert ist oder 1- bis 3 Substituenten aus der Reihe F, Cl, $CF_3$, OH trägt, bedeuten,

mit Y gleich Sauerstoff, Schwefel,

R(2) OH, F, Cl, ($C_1$-$C_{10}$)-Alkylcarbonyloxy (geradkettig oder verzweigt), ($C_1$-$C_{10}$)-Alkyloxy (geradkettig oder verzweigt), Benzyloxy (unsubstituiert oder 1- bis 2-fach substituiert mit F, Cl, $CF_3$, $OCH_3$), Phenylcarbonyloxy, wobei der Phenylrest unsubstituiert ist oder mit 1-3 Substituenten aus der Reihe F, Cl, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1- bis 6-fach substituiert durch F, Cl, Br und/oder $OCH_3$), ($C_2$-$C_{12}$)-Alkenyl (geradkettig oder verzweigt, ein- oder mehrfach ungesättigt, unsubstituiert oder 1-3-fach substituiert durch F, Cl und/oder $OCH_3$), ($C_2$-$C_{10}$)-Alkinyl (geradkettig oder verzweigt, mit einer oder zwei Dreifachbindungen und unsubstituiert), ($C_3$-$C_{10}$)-Alkeninyl (geradkettig oder verzweigt, eine oder mehrere Doppel- und/oder Dreifachbindungen enthaltend und unsubstituiert), ($C_3$-$C_{12}$)-Cycloalkyl (unsubstituiert oder 1-3-fach substituiert durch F, Cl, Br und/oder $CH_3$), ($C_3$-$C_{12}$)-Cycloalkyl-($C_1$-$C_3$)-alkyl (unsubstituiert), Phenyl, Benzyl, Phenyl-($C_2$-$C_3$)-alkyl, Phenoxyphenyl, Phenylthiophenyl, Phenylsulfinylphenyl, Phenylsulfonylphenyl, Phenoxyphenyl-($C_1$-$C_4$)-alkyl, Phenylthiophenyl-($C_1$-$C_2$)-alkyl, Naphthyl, Naphthyl-($C_1$-$C_2$)-alkyl, Biphenylyl, Biphenylyl-($C_1$-$C_2$)-alkyl, Hetaryl, Hetaryl-($C_1$-$C_2$)-alkyl [mit Hetaryl gleich Thiophen, Furan, Pyridin, Oxazol, Isoxazol, Thiazol, Isthiazol, 1,3,4-Oxadiazol, Pyrimidin, Benzothiofuran, Benzothiazol, Benzoxazol, Chinolin und Isochinolin], wobei die genannten aromatischen Ringsysteme und Hetarylsysteme unsubstituiert oder 1- bis 2-fach substituiert sind durch F, Cl, $CF_3$ ($C_1$-$C_{10}$)-Alkyl (geradkettig oder verzweigt, unsubstituiert oder 1- bis 2-fach substituiert durch F und Cl), ($C_3$-$C_6$)-Cycloalkyl (unsubstituiert) oder die aromatischen Ringsysteme oder die Hetarylsysteme substituiert sind durch CN, NR(6)R(7), Z'R(6), worin R(6) ($C_1$-$C_{10}$)-Alkyl (geradkettig oder verzweigt, unsubstituiert oder 1- bis 3-fach substituiert durch F, Cl und/oder $OCH_3$) und Z' Sauerstoff oder Schwefel bedeutet, oder R(6) ($C_3$-$C_{10}$)-Alkenyl (geradkettig oder verzweigt, ein- oder mehrfach ungesättigt, unsubstituiert oder 1- bis 3-fach substituiert durch F, Cl und/oder $OCH_3$) ist oder R(6) Benzyl, Phenyl, Thienylmethyl, Thienyl oder Phenyl-carbonyl bedeutet, wobei die hier für R(6) genannten Ringsysteme ihrerseits unsubstituiert oder 1- bis 2-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R-(6) und R(7) gleich oder verschieden sein können,

R(4) H, ($C_1$-$C_{10}$)-Alkyl (geradkettig oder verzweigt), Benzyl (unsubstituiert oder ein- oder zweifach substituiert durch F, Cl, $CF_3$, ($C_1$-$C_4$)-Alkyl (geradkettig oder verzweigt), $OCH_3$, O-Phenyl oder Phenyl),

R(5) H, ($C_1$-$C_{16}$)-Alkyl (geradkettig oder verzweigt), ($C_2$-$C_{16}$)-Alkenyl (geradkettig oder verzweigt, ein- oder zweifach ungesättigt), ($C_3$-$C_{12}$)-Cycloalkyl, (mono-, bi- oder tricyclisch, ($C_1$-$C_8$)-Alkyl-oxy-($C_2$-$C_8$)alkyl, Phenyl-($C_1$-$C_6$)-Alkyl (geradkettig oder verzweigt), Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Phenyl, wobei die bezüglich R(5) genannten Phenylsysteme unsubstituiert oder ein- bis 3-fach substituiert sind durch Substituenten aus der Gruppe F, Cl, ($C_1$-$C_4$)-Alkyl (geradkettig oder verzweigt), ($C_3$-$C_8$)-Cycloalkyl, ($C_1$-$C_{10}$)-Alkoxy (geradkettig oder verzweigt), Benzyl, Phenethyl, Thiophenyl, $-O-(CH_2)_{1-2}-O-$, oder

R(5) ist 2,2,6,6-Tetramethylpiperidin-4-yl, oder Indol-3-yl-($C_1$-$C_4$)-alkyl,

X Sauerstoff, Schwefel, Sulfinyl oder Sulfonyl,

und ihre Salze mit pharmazeutisch akzeptablen Säuren und in Form von physiologisch hydrolisierbaren und pharmazeutisch akzeptablen Derivaten.

**3.** Verbindung I nach Anspruch 1, in der bedeuten:

R(1) Phenyl, Biphenylyl, Phenoxyphenyl, Benzylphenyl, Benzyloxyphenyl, Phenylthiophenyl, Naphthyl, 1,2,3,4-Tetrahydronaphthyl, Indanyl, Pyridyl,

wobei die genannten Ringsysteme unsubstituiert oder mit 1-3 Substituenten, die gleich oder verschieden sind, substituiert sind, welche F, Cl, ($C_1$-$C_{10}$)-Alkyl (geradkettig oder verzweigt und unsubstituiert oder mit 1-3 F, Cl Atomen substituiert), ($C_3$-$C_{10}$)-Cycloalkyl (mono-, bi- oder multicyclisch und unsubstituiert), Y-($C_1$-$C_{10}$)-Alkyl (geradkettig oder verzweigt), Y-($C_3$-$C_{10}$)-Cycloalkyl (mono-, bi- oder multicyclisch und unsubstituiert), ($C_2$-$C_{10}$)-Alkenyl (geradkettig oder verzweigt, ein- oder mehrfach ungesättigt), Y-($C_2$-$C_{10}$)-Alkenyl (geradkettig oder verzweigt, ein- oder mehrfach ungesättigt), Y-Phenyl, Benzyl, Biphenylyl, wobei die aufgeführten Alkyl- und Alkenylsubstituenten geradkettig oder verzweigt sind und Phenyl unsubstituiert ist oder 1- bis 3 Substituenten aus der Reihe F, Cl, $CF_3$, trägt, bedeuten,

mit Y gleich Sauerstoff, Schwefel,

R(2) OH, F, ($C_1$-$C_6$)-Alkylcarbonyloxy (geradkettig oder verzweigt), ($C_1$-$C_6$)-Alkyloxy (geradkettig oder verzweigt), Benzyloxy (unsubstituiert oder 1-fach substituiert mit F, Cl, $CF_3$), Phenylcarbonyloxy, wobei der Phenylrest unsubstituiert ist oder mit 1-3 Substituenten aus der Reihe F, Cl, Br, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1-3-fach substituiert durch F, Cl und/oder $OCH_3$), ($C_2$-$C_{12}$)-Alkenyl (geradkettig oder verzweigt, ein- oder mehrfach ungesättigt und unsubstituiert), ($C_3$-$C_{12}$)-Cycloalkyl (unsubstituiert), ($C_3$-$C_{10}$)-Cycloalkyl-($C_1$-$C_3$)-alkyl (unsubstituiert), Phenyl, Benzyl, Phenoxyphenyl, Phenylthiophenyl, Phenoxyphenyl-($C_1$)-alkyl, Phenylthiophenyl-($C_1$)-alkyl, Naphthyl-($C_1$)-alkyl, Biphenylyl, Biphenylyl-($C_1$)-alkyl, Hetaryl, Hetarylmethyl mit Hetaryl gleich Thiophen, Furan, Pyridin, Oxazol, Isoxazol, Thiazol, Isthiazol, Pyrimidin, Benzothiazol und Benzoxazol), wobei die genannten aromatischen Ringsysteme und Hetarylsysteme unsubstituiert oder 1- bis 2-fach substituiert sind durch F, Cl, $CF_3$, ($C_1$-$C_{10}$)-Alkyl (geradkettig oder verzweigt und unsubstituiert), ($C_3$-$C_6$)-Cycloalkyl (unsubstituiert) oder die aromatischen Ringsysteme oder die Hetarylsysteme substituiert sind durch CN, NR(6)R(7), Z'R(6), worin R(6) ($C_1$-$C_{10}$)-Alkyl (geradkettig oder verzweigt und unsubstituiert) und Z' gleich Sauerstoff oder Schwefel bedeutet oder R(6) ($C_3$-$C_6$)-Alkenyl (geradkettig oder verzweigt, ein- oder mehrfach ungesättigt und unsubstituiert) oder R(6) Benzyl, Phenyl, Thienylmethyl oder Thienyl bedeutet, wobei die hier für R(6) genannten Ringsysteme ihrerseits unsubstituiert oder 1- bis 2-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R(6) und R(7) gleich oder verschieden sein können,

R(4) H, ($C_1$-$C_{10}$)-Alkyl (geradkettig oder verzweigt), Benzyl (unsubstituiert oder ein- oder zweifach substituiert durch F, Cl, $CF_3$, ($C_1$-$C_4$)-Alkyl (geradkettig oder verzweigt), $OCH_3$, O-Phenyl oder Phenyl),

R(5) H, ($C_1$-$C_{12}$)-Alkyl (geradkettig oder verzweigt), ($C_2$-$C_{12}$)-Alkenyl (geradkettig oder verzweigt,

ein- oder zweifach ungesättigt), ($C_3$-$C_{12}$)-Cycloalkyl, (mono-, bi- oder tricyclisch), Phenyl-($C_1$-$C_6$)-Alkyl (geradkettig oder verzweigt), Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Phenyl, wobei die bezüglich R(5) genannten Phenylsysteme unsubstituiert oder ein- bis 3-fach substituiert sind durch Substituenten aus der Gruppe F, Cl, ($C_1$-$C_4$)-Alkyl (geradkettig oder verzweigt), ($C_3$-$C_6$)-Cycloalkyl, ($C_1$-$C_{10}$)-Alkoxy (geradkettig oder verzweigt), Benzyl, Phenethyl,

X Sauerstoff oder Schwefel,

und ihre Salze mit pharmazeutisch akzeptablen Säuren und in Form von physiologisch hydrolysierbaren und pharmazeutisch akzeptablen Derivaten.

**4.** Verbindung I nach Anspruch 1, in der

R(1) Phenyl, Biphenylyl, Phenoxyphenyl, Benzylphenyl, Benzyloxyphenyl, Phenylthiophenyl, Naphthyl, Pyridyl,

wobei die genannten Ringsysteme unsubstituiert oder mit 1-2 Substituenten, die gleich oder verschieden sind, substituiert sind, welche F, Cl, ($C_1$-$C_{10}$)-Alkyl (geradkettig oder verzweigt und unsubstituiert), ($C_3$-$C_{10}$)-Cycloalkyl (mono-, bi- oder multicyclisch und unsubstituiert), Y-($C_1$-$C_{10}$)-Alkyl (geradkettig oder verzweigt), Y-($C_3$-$C_{10}$)-Cycloalkyl (mono-, bi- oder multicyclisch und unsubstituiert), ($C_2$-$C_{10}$)-Alkenyl (geradkettig oder verzweigt, ein- oder zweifach ungesättigt), Y-($C_2$-$C_{10}$)-Alkenyl (geradkettig oder verzweigt, ein- oder zweifach ungesättigt), Y-Phenyl, Benzyl, Biphenylyl, wobei die aufgeführten Alkyl- und Alkenylsubstituenten geradkettig oder verzweigt sind und Phenyl unsubstituiert ist oder 1- bis 2 Substituenten aus der Reihe F, Cl, $CF_3$ trägt), bedeuten,
mit Y gleich Sauerstoff, Schwefel,

R(3) ($C_1$-$C_{10}$)-Alkyl (geradkettig oder verzweigt, unsubstituiert), ($C_3$-$C_{12}$)-Alkenyl (geradkettig oder verzweigt, ein- oder zweifach ungesättigt und unsubstituiert), ($C_3$-$C_6$)-Cycloalkyl (unsubstituiert), Phenyl, Benzyl, Phenoxyphenyl, Phenylthiophenyl, Phenoxyphenyl-($C_1$)-alkyl, Phenylthiophenyl-($C_1$)-alkyl, Naphthyl-($C_1$)-alkyl, Biphenylyl, Biphenylyl-($C_1$)-alkyl, Hetaryl, Hetarylmethyl (mit Hetaryl gleich Thiophen, Pyridin, Oxazol, Isoxazol), wobei die genannten aromatischen Ringsysteme und Hetarylsysteme unsubstituiert oder 1-bis 2-fach substituiert sind durch F, Cl, $CF_3$, ($C_1$-$C_{10}$)-Alkyl (geradkettig oder verzweigt und unsubstituiert), ($C_3$-$C_6$)-Cycloalkyl (unsubstituiert) oder die aromatischen Ringsysteme oder die Hetarylsysteme unsubstituiert oder substituiert sind durch CN, NR(6)R(7), Z'R(6), worin R(6) ($C_1$-$C_{10}$)-Alkyl (geradkettig oder verzweigt und unsubstituiert) und Z' Sauerstoff oder Schwefel bedeutet oder R(6) ($C_3$-$C_6$)-Alkenyl (geradkettig oder verzweigt, ein- oder mehrfach ungesättigt und unsubstituiert) oder R(6) Benzyl oder Phenyl, bedeutet, wobei die hier für R(6) genannten Ringsysteme ihrerseits unsubstituiert oder 1- bis 2-fach substituiert sind durch F, Cl, $CF_3$, ($C_1$-$C_4$)-Alkyl und/oder ($C_1$-$C_4$)-Alkoxy und R(7) entweder Wasserstoff oder wie R-(6) definiert ist,

R(4) H

R(5) H, ($C_1$-$C_{10}$)-Alkyl (geradkettig oder verzweigt), ($C_2$-$C_{10}$)-Alkenyl (geradkettig oder verzweigt, ein- oder zweifach ungesättigt), ($C_3$-$C_{12}$)-Cycloalkyl, (mono-, bi- oder tricyclisch), Phenyl-($C_1$-$C_6$)-Alkyl (geradkettig oder verzweigt), Phenyl($C_2$-$C_6$)-Alkenyl (geradkettig oder verzweigt, ein- oder zweifach ungesättigt), Diphenyl-($C_1$-$C_6$)-alkyl (geradkettig oder verzweigt), Phenyl, wobei die bezüglich R(5) genannten Phenylsysteme unsubstituiert oder ein- bis 2-fach substituiert sind durch Substituenten aus der Gruppe F, Cl, ($C_1$-$C_4$)-Alkyl (geradkettig oder verzweigt), ($C_3$-$C_6$)-Cycloalkyl, ($C_1$-$C_4$)-Alkoxy (geradkettig oder verzweigt), Benzyl, Phenethyl,
und

X Sauerstoff oder Schwefel,

und ihre Salze mit pharmazeutisch akzeptablen Säuren und in Form von physiologisch hydrolysierbaren und pharmazeutisch akzeptablen Derivaten.

**5.** Verfahren zum Herstellen einer Verbindung I nach Anspruch 1, dadurch gekennzeichnet, daß man eine Verbindung der Formel (II)

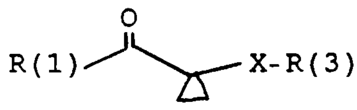 (II)

in welcher R(1), R(3) und X die genannten Bedeutungen haben, mit einem Schwefelylid der Formel III,

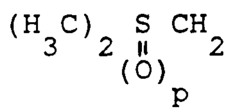 III,

worin p Null oder 1 bedeutet,

zu einer Verbindung der Formel IV

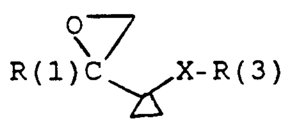 (IV)

umsetzt,
und anschließend die Verbindung IV mit einem Nucleophil der Formel MNR(4)R(5) umsetzt, in welcher R(4) und R(5) die genannten Bedeutungen hat und M Wasserstoff oder ein Metalläquivalent ist, wobei eine Verbindung I mit R(2) = OH entsteht, und falls gewünscht, diese Verbindung I acyliert oder alkyliert und gegebenenfalls am Schwefel einer Thioethergruppe zum Sulfoxid oder Sulfon oxidiert und gegebenenfalls die Verbindung I mit R(2) = OH in eine Verbindung mit R(2) = F, Cl oder Brom überführt und eine so erhaltene Verbindung der Formel (I) in Form der freien Base oder eines Säureadditionssalzes isoliert.

**6.** Verwendung einer Verbindung der Formel I nach Anspruch 1 zur Herstellung eines Antimykotikums.

**7.** Verwendung einer Verbindung der Formel I nach Anspruch 1 als Antimykotikum.

**8.** Antimykotische Zubereitung, gekennzeichnet durch einen wirksamen Gehalt einer Verbindung der Formel I nach Anspruch 1 und pharmazeutisch annehmbaren Zusatzstoffen.

**9.** Verwendung einer Verbindung der Formel I nach Anspruch 1 als Wachstumshemmer der pathogenen Phase dimorpher Hefen.

**Patentansprüche für folgende Vertragsstaaten : ES, GR**

**1.** Verfahren zum Herstellen einer Verbindung I der Formel I

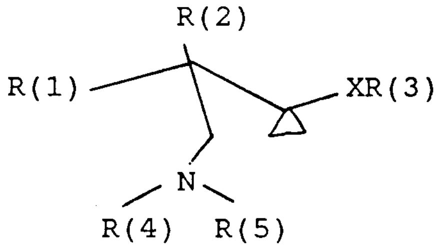 I

in welcher bedeuten:

R(1) t-Butyl, Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche

F, Cl, Br, J, ($C_1$-$C_{18}$)-Alkyl (geradkettig oder verzweigt und unsubstituiert oder mit 1-9 F-, Cl-Atomen substituiert), ($C_3$-$C_{18}$)-Cycloalkyl [mono-, bi- oder multicyclisch, unsubstituiert oder ein- oder zweifach substituiert mit ($C_1$-$C_4$)-Alkyl (geradkettig oder verzweigt), ($C_1$-$C_4$)-Alkoxy (geradkettig oder verzweigt), $CF_3$, F, Cl, Br, OH Y-($C_1$-$C_{18}$)-Alkyl (geradkettig oder verzweigt), Y-($C_3$-$C_{18}$)-Cycloalkyl [mono-, bi- oder multicyclisch, unsubstituiert oder wie oben angegeben substituiert], ($C_2$-$C_{15}$)-Alkenyl (geradkettig oder verzweigt, ein- oder mehrfach ungesättigt), Y-($C_2$-$C_{15}$)-Alkenyl (geradkettig oder verzweigt, ein- oder mehrfach ungesättigt), Y-Phenyl, Y-($C_1$-$C_2$)-Alkyl-phenyl, Phenyl-($C_1$-$C_2$)-alkyl, Biphenylyl, CN, $NO_2$, $CO_2Z$ (mit Z gleich Phenyl, ($C_1$-$C_{15}$)-Alkyl, ($C_2$-$C_{15}$)-Alkenyl, wobei die aufgeführten Alkyl- und Alkenylsubstituenten geradkettig oder verzweigt sind und Phenyl unsubstituiert ist oder 1-3 Substituenten aus der Reihe F, Cl, $CF_3$, OH trägt), bedeuten,

mit Y gleich Sauerstoff, Schwefel, Sulfinyl, Sulfonyl,

R(2) OH, F, Cl, Br, ($C_1$-$C_{10}$)-Alkylcarbonyloxy (geradkettig oder verzweigt), ($C_1$-$C_{10}$)-Alkyloxy (geradkettig oder verzweigt), Benzyloxy (unsubstituiert oder 1-2fach substituiert mit F, Cl, Br, $CF_3$, $OCH_3$), Phenylcarbonyloxy, wobei der Phenylrest unsubstituiert ist oder mit 1-3 Substituenten aus der Reihe F, Cl, Br, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1-9fach substituiert durch F, Cl, Br, J und/oder $OCH_3$), ($C_2$-$C_{12}$)-Alkenyl (geradkettig oder verzweigt, ein- oder mehrfach ungesättigt, in Form des reinen E- oder Z-Diastereomeren oder als E/Z-Diastereomerengemisch, unsubstituiert oder 1- bis 9-fach substituiert durch F, Cl, Br, J und/oder $OCH_3$), ($C_2$-$C_{12}$)-Alkinyl (geradkettig oder verzweigt, mit einer oder mehreren Dreifachbindungen, unsubstituiert oder 1- bis 9-fach substituiert durch F, Cl, Br, J und/oder $OCH_3$), ($C_3$-$C_{12}$)-Alkeninyl (geradkettig oder verzweigt, eine oder mehrere Doppel- und/oder Dreifachbindungen enthaltend, in Form des reinen E- oder Z-Diastereomeren oder als E/Z-Diastereomerengemisch, unsubstituiert oder 1- bis 9-fach substituiert durch F, Cl, Br, J und/oder $OCH_3$), ($C_3$-$C_{12}$)-Cycloalkyl (unsubstituiert oder 1- bis 9-fach substituiert durch F, Cl, Br, J, $OCH_3$ und/oder $CH_3$), ($C_3$-$C_{12}$)-Cycloalkyl-($C_1$-$C_3$)-alkyl (unsubstituiert oder 1-9-fach substituiert durch F, Cl, Br und/oder $CH_3$), Phenyl, Benzyl, Phenyl-($C_2$-$C_4$)-alkyl, Phenoxyphenyl, Phenylthiophenyl, Phenylsulfinylphenyl, Phenylsulfonylphenyl, Phenoxyphenyl-($C_1$-$C_4$)-alkyl, Phenylthiophenyl-($C_1$-$C_4$)-alkyl, Naphthyl, Naphthyl-($C_1$-$C_4$)-alkyl, Biphenylyl, Biphenyl-($C_1$-$C_4$)-alkyl, Hetaryl, Hetaryl-($C_1$-$C_2$)-alkyl (mit Hetaryl gleich Thiophen, Furan, Pyridin, Oxazol, Isoxazol, Thiazol, Isthiazol, Pyrrol, 1,2,4-Oxadiazol, 1,3,4-Oxadiazol, Pyrimidin, Pyrazin, Benzothiofuran, Benzothiazol, Benzoxazol, Indol, Chinolin und Isochinolin), wobei die genannten aromatischen Ringsysteme und Hetarylsysteme unsubstituiert oder 1- bis 3-fach substituiert sind durch F, Cl, Br, $CF_3$, ($C_1$-$C_{10}$)-Alkyl (geradkettig oder verzweigt, unsubstituiert oder 1- bis 3-fach substituiert durch F, Cl und/oder $OCH_3$), ($C_3$-$C_6$)-Cycloalkyl (unsubstituiert), oder die aromatischen Ringsysteme oder die Hetarylsysteme substituiert sind durch Phenyl (unsubstituiert oder 1- bis 3-fach substituiert durch F, Cl, $OCH_3$), CN, OH, $NH_2$, NR(6)R(7), $NO_2$, Z'R(6), worin R(6) ($C_1$-$C_{10}$)-Alkyl (geradkettig oder verzweigt, unsubstituiert oder 1- bis 9-fach substituiert durch F, Cl und/oder $OCH_3$) und Z' gleich Sauerstoff oder Schwefel ist, oder R(6) ($C_3$-$C_{12}$)-Alkenyl (geradkettig oder verzweigt, ein- oder mehrfach ungesättigt, unsubstituiert oder 1- bis 3-fach substituiert durch F, Cl und/oder $OCH_3$) ist, oder R(6) Benzyl, Biphenylylmethyl, Naphthylmethyl, Phenyl, Thienylmethyl, Thienyl, ($C_1$-$C_{10}$)-Alkylcarbonyl (geradkettig oder verzweigt) oder Phenyl-carbonyl ist, wobei die hier für R(6) genannten Ringsysteme ihrerseits unsubstituiert oder 1- bis 3-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R(6) und R(7) gleich oder verschieden sein können,

R(4) H, ($C_1$-$C_{18}$)-Alkyl (geradkettig oder verzweigt), ($C_2$-$C_{10}$)-Alkenyl (geradkettig oder verzweigt,

ein- oder mehrfach ungesättigt), Benzyl (unsubstituiert oder ein- oder mehrfach substituiert durch F, Cl, Br, $CF_3$, ($C_1$-$C_4$)-Alkyl (geradkettig oder verzweigt), $OCH_3$, O-Phenyl oder Phenyl),

R(5) H, ($C_1$-$C_{18}$)-Alkyl (geradkettig oder verzweigt), ($C_2$-$C_{18}$)-Alkenyl (geradkettig oder verzweigt, ein- oder mehrfach ungesättigt), ($C_3$-$C_{12}$)-Cycloalkyl, (mono-, bi- oder tricyclisch,wie z.B. Cyclohexyl, Norbornyl oder Adamantyl) ($C_1$-$C_8$)-Alkyl-oxy-($C_2$-$C_{10}$)-alkyl, Phenyl-($C_1$-$C_6$)-Alkyl (geradkettig oder verzweigt), Phenyl-oxy-($C_1$-$C_6$)-alkyl, Phenyl-thio-($C_1$-$C_6$)-alkyl, Phenyl-($C_2$-$C_6$)-Alkenyl (geradkettig oder verzweigt, ein- oder zweifach ungesättigt), Diphenyl-($C_1$-$C_6$)-alkyl (geradkettig oder verzweigt), Thienyl, Thienyl-methyl, Phenyl, wobei die bezüglich R(5) genannten Phenyl- oder Thienylsysteme unsubstituiert oder ein- bis 3-fach substituiert sind durch Substituenten aus der Gruppe F, Cl, Br, ($C_1$-$C_{10}$)-Alkyl (geradkettig oder verzweigt), ($C_3$-$C_8$)-Cycloalkyl, OH, SH, ($C_1$-$C_{10}$)-Alkoxy (geradkettig oder verzweigt), Phenyl, Benzyl, Phenethyl, Thiophenyl, $C_aF_{a+1}$ mit a = 1-6), -O $(CH_2)_{1-2}$O-,

oder

R(5) ist ein Piperidin-4-yl-Rest, der unsubstituiert ist oder durch 1- bis 4 Methylgruppen substituiert ist,

oder

R(5) ist ein Indol-3-yl-($C_1$-$C_4$)-Alkylrest (geradkettig oder verzweigt)

R(4) mit R(5) eine Kette aus $(CH_2)$m-Einheiten mit m = 4-6 bilden, die durch O, S oder N-Atom unterbrochen sein kann, wobei H als weiteren Bindungspartner ein H-Atom, eine $CH_3$, Phenyl-, Benzyl oder Phenethylgruppe trägt und der so gebildete Heterocyclus an den $CH_2$-Einheiten unsubstituiert ist oder 1- bis 4 $CH_3$-Gruppen als Substituenten trägt) und

X Sauerstoff, Schwefel, Sulfinyl oder Sulfonyl,

und von ihren Salzen mit pharmazeutisch akzeptablen Säuren und von physiologisch hydrolysierbaren und pharmazeutisch akzeptablen Derivaten,

dadurch gekennzeichnet, daß man eine Verbindung der Formel (II)

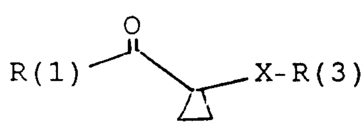

(II)

in welcher R(1), R(3) und X die genannten Bedeutungen haben, mit einem Schwefelylid der Formel III,

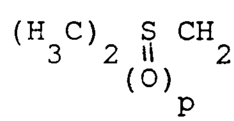

III,

worin p Null oder 1 bedeutet,

zu einer Verbindung der Formel IV

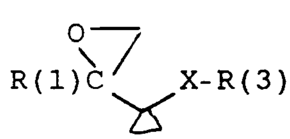

(IV)

umsetzt,
und anschließend die Verbindung IV mit einem Nucleophil der Formel MNR(4)R(5) umsetzt, in welcher R(4) und R(5) die genannten Bedeutungen hat und M Wasserstoff oder ein Metalläquivalent ist, wobei eine Verbindung I mit R(2) = OH entsteht, und falls gewünscht, diese Verbindung I acyliert oder alkyliert und gegebenenfalls am Schwefel einer Thioethergruppe zum Sulfoxid oder Sulfon oxidiert und

gegebenenfalls die Verbindung I mit R(2) = OH in eine Verbindung mit R(2) = F, Cl oder Brom überführt und eine so erhaltene Verbindung der Formel (I) in Form der freien Base oder eines Säureadditionssalzes isoliert.

**2.** Verfahren nach Anspruch 1, dadurch gekennzeichnet, daß bedeuten:

R(1) Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Pyridyl,

wobei die genannten Ringsysteme unsubstituiert oder mit 1-3 Substituenten, die gleich oder verschieden sind, substituiert sind, welche

F, Cl, ($C_1$-$C_{12}$)-Alkyl (geradkettig oder verzweigt und unsubstituiert oder mit 1-6 F-, Cl-Atomen substituiert), ($C_3$-$C_{18}$)-Cycloalkyl [mono, bi- oder multicyclisch, unsubstituiert oder einfach substituiert mit ($C_1$-$C_4$)-Alkyl (geradkettig oder verzweigt), ($C_1$-$C_4$)-Alkoxy (geradkettig oder verzweigt), $CF_3$, F, Cl, OH], Y-($C_1$-$C_{10}$)-Alkyl (geradkettig oder verzweigt), Y-($C_3$-$C_{10}$)-Cycloalkyl [mono-, bi- oder multicyclisch, unsubstituiert oder wie oben angegeben substituiert], ($C_2$-$C_{10}$)-Alkenyl (geradkettig oder verzweigt, ein- oder mehrfach ungesättigt), Y-($C_2$-$C_{10}$)-Alkenyl (geradkettig oder verzweigt, ein- oder mehrfach ungesättigt), Y-Phenyl, Y-($C_1$-$C_2$)-Alkyl-phenyl, Phenyl-($C_1$-$C_2$)-alkyl, Biphenylyl, wobei die aufgeführten Alkyl- und Alkenylsubstituenten geradkettig oder verzweigt sind und Phenyl unsubstituiert ist oder 1- bis 3 Substituenten aus der Reihe F, Cl, $CF_3$, OH trägt, bedeuten,

mit Y gleich Sauerstoff, Schwefel,

R(2) OH, F, Cl, ($C_1$-$C_{10}$)-Alkylcarbonyloxy (geradkettig oder verzweigt), ($C_1$-$C_{10}$)-Alkyloxy (geradkettig oder verzweigt), Benzyloxy (unsubstituiert oder 1- bis 2-fach substituiert mit F, Cl, $CF_3$, $OCH_3$), Phenylcarbonyloxy, wobei der Phenylrest unsubstituiert ist oder mit 1-3 Substituenten aus der Reihe F, Cl, $CF_3$, ($C_1$-$C_4$)-Alkyl (geradkettig oder verzweigt) substituiert ist,

R(3) ($C_1$-$C_{18}$)-Alkyl (geradkettig oder verzweigt, unsubstituiert ist oder 1- bis 6-fach substituiert durch F, Cl, Br und/oder $OCH_3$), ($C_2$-$C_{12}$)-Alkenyl (geradkettig oder verzweigt, ein- oder mehrfach ungesättigt, unsubstituiert oder 1-3-fach substituiert durch F, Cl und/oder $OCH_3$), ($C_2$-$C_{10}$)-Alkinyl (geradkettig oder verzweigt, mit einer oder zwei Dreifachbindungen und unsubstituiert), ($C_3$-$C_{10}$)-Alkeninyl (geradkettig oder verzweigt, eine oder mehrere Doppel- und/oder Dreifachbindungen enthaltend und unsubstituiert), ($C_3$-$C_{12}$)-Cycloalkyl (unsubstituiert oder 1-3-fach substituiert durch F, Cl, Br und/oder $CH_3$), ($C_3$-$C_{12}$)-Cycloalkyl-($C_1$-$C_3$)-alkyl (unsubstituiert), Phenyl, Benzyl, Phenyl-($C_2$-$C_3$)-alkyl, Phenoxyphenyl, Phenylthiophenyl, Phenylsulfinylphenyl, Phenylsulfonylphenyl, Phenoxyphenyl-($C_1$-$C_4$)-alkyl, Phenylthiophenyl-($C_1$-$C_2$)-alkyl, Naphthyl, Naphthyl-($C_1$-$C_2$)-alkyl, Biphenylyl, Biphenylyl-($C_1$-$C_2$)-alkyl, Hetaryl, Hetaryl-($C_1$-$C_2$)-alkyl [mit Hetaryl gleich Thiophen, Furan, Pyridin, Oxazol, Isoxazol, Thiazol, Isthiazol, 1,3,4-Oxadiazol, Pyrimidin, Benzothiofuran, Benzothiazol, Benzoxazol, Chinolin und Isochinolin], wobei die genannten aromatischen Ringsysteme und Hetarylsysteme unsubstituiert oder 1- bis 2-fach substituiert sind durch F, Cl, $CF_3$ ($C_1$-$C_{10}$)-Alkyl (geradkettig oder verzweigt, unsubstituiert oder 1- bis 2-fach substituiert durch F und Cl), ($C_3$-$C_6$)-Cycloalkyl (unsubstituiert) oder die aromatischen Ringsysteme oder die Hetarylsysteme substituiert sind durch CN, NR(6)R(7), Z'R(6), worin R(6) ($C_1$-$C_{10}$)-Alkyl (geradkettig oder verzweigt, unsubstituiert oder 1- bis 3-fach substituiert durch F, Cl und/oder $OCH_3$) und Z' Sauerstoff oder Schwefel bedeutet, oder R(6) ($C_3$-$C_{10}$)-Alkenyl (geradkettig oder verzweigt, ein- oder mehrfach ungesättigt, unsubstituiert oder 1- bis 3-fach substituiert durch F, Cl und/oder $OCH_3$) ist oder R(6) Benzyl, Phenyl, Thienylmethyl, Thienyl oder Phenyl-carbonyl bedeutet, wobei die hier für R(6) genannten Ringsysteme ihrerseits unsubstituiert oder 1- bis 2-fach substituiert sind durch F, Cl, $CF_3$, ($C_1$-$C_4$)-Alkyl und/oder ($C_1$-$C_4$)-Alkoxy und R(7) entweder Wasserstoff oder wie R(6) definiert ist, wobei R(6) und R(7) gleich oder verschieden sein können,

R(4) H, ($C_1$-$C_{10}$)-Alkyl (geradkettig oder verzweigt), Benzyl (unsubstituiert oder ein- oder zweifach substituiert durch F, Cl, $CF_3$, ($C_1$-$C_4$)-Alkyl (geradkettig oder verzweigt), $OCH_3$, O-Phenyl oder Phenyl),

R(5) H, ($C_1$-$C_{16}$)-Alkyl (geradkettig oder verzweigt), ($C_2$-$C_{16}$)-Alkenyl (geradkettig oder verzweigt,

ein- oder zweifach ungesättigt), $(C_3-C_{12})$-Cycloalkyl, (mono-, bi- oder tricyclisch), $(C_1-C_8)$-Alkyl-oxy-$(C_2-C_8)$-alkyl, Phenyl-$(C_1-C_6)$-Alkyl (geradkettig oder verzweigt), Phenyl-$(C_2-C_6)$-Alkenyl (geradkettig oder verzweigt, ein- oder zweifach ungesättigt), Diphenyl-$(C_1-C_6)$-alkyl (geradkettig oder verzweigt), Phenyl, wobei die bezüglich R(5) genannten Phenylsysteme unsubstituiert oder ein- bis 3-fach substituiert sind durch Substituenten aus der Gruppe F, Cl, $(C_1-C_4)$-Alkyl (geradkettig oder verzweigt), $(C_3-C_8)$-Cycloalkyl, $(C_1-C_{10})$-Alkoxy (geradkettig oder verzweigt), Benzyl, Phenethyl, Thiophenyl, $-O-(CH_2)_{1-2}-O-$,
oder

R(5) ist 2,2,6,6-Tetramethylpiperidin-4-yl, oder Indol-3-yl-$(C_1-C_4)$-alkyl,

X Sauerstoff, Schwefel, Sulfinyl oder Sulfonyl.

**3.** Verfahren nach Anspruch 1, dadurch gekennzeichnet, daß bedeuten:

R(1) Phenyl, Biphenylyl, Phenoxyphenyl, Benzylphenyl, Benzyloxyphenyl, Phenylthiophenyl, Naphthyl, 1,2,3,4-Tetrahydronaphthyl, Indanyl, Pyridyl,

wobei die genannten Ringsysteme unsubstituiert oder mit 1-3 Substituenten, die gleich oder verschieden sind, substituiert sind, welche F, Cl, $(C_1-C_{10})$-Alkyl (geradkettig oder verzweigt und unsubstituiert oder mit 1-3 F, Cl Atomen substituiert), $(C_3-C_{10})$-Cycloalkyl (mono-, bi- oder multicyclisch und unsubstituiert, wie z.B. Norbornyl-, Adamantyl, Dekahydronaphthyl), Y-$(C_1-C_{10})$-Alkyl (geradkettig oder verzweigt), Y-$(C_3-C_{10})$-Cycloalkyl (mono-, bi- oder multicyclisch und unsubstituiert), $(C_2-C_{10})$-Alkenyl (geradkettig oder verzweigt, ein- oder mehrfach ungesättigt), Y-$(C_2-C_{10})$-Alkenyl (geradkettig oder verzweigt, ein- oder mehrfach ungesättigt), Y-Phenyl, Benzyl, Biphenylyl, wobei die aufgeführten Alkyl- und Alkenylsubstituenten geradkettig oder verzweigt sind und Phenyl unsubstituiert ist oder 1- bis 3 Substituenten aus der Reihe F, Cl, $CF_3$, trägt, bedeuten,

mit Y gleich Sauerstoff, Schwefel,

R(2) OH, F, $(C_1-C_6)$-Alkylcarbonyloxy (geradkettig oder verzweigt), $(C_1-C_6)$-Alkyloxy (geradkettig oder verzweigt), Benzyloxy (unsubstituiert oder 1-fach substituiert mit F, Cl, $CF_3$), Phenylcarbonyloxy, wobei der Phenylrest unsubstituiert ist oder mit 1-3 Substituenten aus der Reihe F, Cl, Br, $CF_3$, $(C_1-C_4)$-Alkyl (geradkettig oder verzweigt) substituiert ist,

R(3) $(C_1-C_{18})$-Alkyl (geradkettig oder verzweigt, unsubstituiert ist oder 1-3-fach substituiert durch F, Cl und/oder $OCH_3$), $(C_2-C_{12})$-Alkenyl (geradkettig oder verzweigt, ein- oder mehrfach ungesättigt und unsubstituiert), $(C_3-C_{12})$-Cycloalkyl (unsubstituiert), $(C_3-C_{10})$-Cycloalkyl-$(C_1-C_3)$-alkyl (unsubstituiert), Phenyl, Benzyl, Phenoxyphenyl, Phenylthiophenyl, Phenoxyphenyl-$(C_1)$-alkyl, Phenylthiophenyl-$(C_1)$-alkyl, Naphthyl-$(C_1)$-alkyl, Biphenylyl, Biphenylyl-$(C_1)$-alkyl, Hetaryl, Hetarylmethyl mit Hetaryl gleich Thiophen, Furan, Pyridin, Oxazol, Isoxazol, Thiazol, Isthiazol, Pyrimidin, Benzothiazol und Benzoxazol), wobei die genannten aromatischen Ringsysteme und Hetarylsysteme unsubstituiert oder 1- bis 2-fach substituiert sind durch F, Cl, $CF_3$, $(C_1-C_{10})$-Alkyl (geradkettig oder verzweigt und unsubstituiert), $(C_3-C_6)$-Cycloalkyl (unsubstituiert) oder die aromatischen Ringsysteme oder die Hetarylsysteme substituiert sind durch CN, NR(6)R(7), Z'R(6), worin R(6) $(C_1-C_{10})$-Alkyl (geradkettig oder verzweigt und unsubstituiert) und Z' gleich Sauerstoff oder Schwefel bedeutet oder R(6) $(C_3-C_6)$-Alkenyl (geradkettig oder verzweigt, ein- oder mehrfach ungesättigt und unsubstituiert) oder R(6) Benzyl, Phenyl, Thienylmethyl oder Thienyl bedeutet, wobei die hier für R(6) genannten Ringsysteme ihrerseits unsubstituiert oder 1- bis 2-fach substituiert sind durch F, Cl, $CF_3$, $(C_1-C_4)$-Alkyl und/oder $(C_1-C_4)$-Alkoxy und R-(7) entweder Wasserstoff oder wie R(6) definiert ist, wobei R(6) und R(7) gleich oder verschieden sein können,

R(4) H, $(C_1-C_{10})$-Alkyl (geradkettig oder verzweigt), Benzyl (unsubstituiert oder ein- oder zweifach substituiert durch F, Cl, $CF_3$, $(C_1-C_4)$-Alkyl (geradkettig oder verzweigt), $OCH_3$, O-Phenyl oder Phenyl),

R(5) H, $(C_1-C_{12})$-Alkyl (geradkettig oder verzweigt), $(C_2-C_{12})$-Alkenyl (geradkettig oder verzweigt, ein- oder zweifach ungesättigt, $(C_3-C_{12})$-Cycloalkyl, (mono-, bi- oder tricyclisch), Phenyl-$(C_1-C_6)$-Alkyl (geradkettig oder verzweigt), Phenyl-$(C_2-C_6)$-Alkenyl (geradkettig oder verzweigt, ein- oder zweifach ungesättigt), Diphenyl-$(C_1-C_6)$-alkyl (geradkettig oder verzweigt), Phenyl, wobei die bezüglich R(5) genannten Phenylsysteme unsubstituiert oder ein- bis 3-fach substituiert sind durch Substituenten aus der Gruppe F, Cl, $(C_1-C_4)$-Alkyl (geradkettig

oder verzweigt), $(C_3\text{-}C_6)$-Cycloalkyl, $(C_1\text{-}C_{10})$-Alkoxy (geradkettig oder verzweigt), Benzyl, Phenethyl,

X Sauerstoff oder Schwefel.

**4.** Verfahren nach Anspruch 1, dadurch gekennzeichnet, daß bedeuten:

R(1) Phenyl, Biphenylyl, Phenoxyphenyl, Benzylphenyl, Benzyloxyphenyl, Phenylthiophenyl, Naphthyl, Pyridyl, wobei die genannten Ringsysteme unsubstituiert oder mit 1-2 Substituenten, die gleich oder verschieden sind, substituiert sind, welche F, Cl, $(C_1\text{-}C_{10})$-Alkyl (geradkettig oder verzweigt und unsubstituiert), $(C_3\text{-}C_{10})$-Cycloalkyl (mono-, bi- oder multicyclisch und unsubstituiert), Y-$(C_1\text{-}C_{10})$-Alkyl (geradkettig oder verzweigt), Y-$(C_3\text{-}C_{10})$-Cycloalkyl (mono-, bi- oder multicyclisch und unsubstituiert), $(C_2\text{-}C_{10})$-Alkenyl (geradkettig oder verzweigt, ein- oder zweifach ungesättigt), Y-$(C_2\text{-}C_{10})$-Alkenyl (geradkettig oder verzweigt, ein- oder zweifach ungesättigt), Y-Phenyl, Benzyl, Biphenylyl, wobei die aufgeführten Alkyl- und Alkenylsubstituenten geradkettig oder verzweigt sind und Phenyl unsubstituiert ist oder 1- bis 2 Substituenten aus der Reihe F, Cl, $CF_3$ trägt), bedeuten,
mit Y gleich Sauerstoff, Schwefel,

R(3) $(C_1\text{-}C_{10})$-Alkyl (geradkettig oder verzweigt, unsubstituiert), $(C_3\text{-}C_{12})$-Alkenyl (geradkettig oder verzweigt, ein- oder zweifach ungesättigt und unsubstituiert), $(C_3\text{-}C_6)$-Cycloalkyl (unsubstituiert), Phenyl, Benzyl, Phenoxyphenyl, Phenylthiophenyl, Phenoxyphenyl-$(C_1)$-alkyl, Phenylthiophenyl-$(C_1)$-alkyl, Naphthyl-$(C_1)$alkyl, Biphenylyl, Biphenylyl-$(C_1)$-alkyl, Hetaryl, Hetarylmethyl (mit Hetaryl gleich Thiophen, Pyridin, Oxazol, Isoxazol), wobei die genannten aromatischen Ringsysteme und Hetarylsysteme unsubstituiert oder 1-bis 2-fach substituiert sind durch F, Cl, $CF_3$, $(C_1\text{-}C_{10})$-Alkyl (geradkettig oder verzweigt und unsubstituiert), $(C_3\text{-}C_6)$-Cycloalkyl (unsubstituiert) oder die aromatischen Ringsysteme oder die Hetarylsysteme unsubstituiert oder substituiert sind durch CN, NR(6)R(7), Z'R(6), worin R(6) $(C_1\text{-}C_{10})$-Alkyl (geradkettig oder verzweigt und unsubstituiert) und Z' Sauerstoff oder Schwefel bedeutet oder R(6) $(C_3\text{-}C_6)$-Alkenyl (geradkettig oder verzweigt, ein- oder mehrfach ungesättigt und unsubstituiert) oder R(6) Benzyl oder Phenyl, bedeutet, wobei die hier für R(6) genannten Ringsysteme ihrerseits unsubstituiert oder 1- bis 2-fach substituiert sind durch F, Cl, $CF_3$, $(C_1\text{-}C_4)$-Alkyl und/oder $(C_1\text{-}C_4)$-Alkoxy und R(7) entweder Wasserstoff oder wie R-(6) definiert ist,

R(4) H

R(5) H, $(C_1\text{-}C_{10})$-Alkyl (geradkettig oder verzweigt), $(C_2\text{-}C_{10})$-Alkenyl (geradkettig oder verzweigt, ein- oder zweifach ungesättigt, $(C_3\text{-}C_{12})$-Cycloalkyl, (mono-, bi- oder tricyclisch), Phenyl-$(C_1\text{-}C_6)$-Alkyl (geradkettig oder verzweigt), Phenyl-$(C_2\text{-}C_6)$-Alkenyl (geradkettig oder verzweigt, ein- oder zweifach ungesättigt), Diphenyl-$(C_1\text{-}C_6)$-alkyl (geradkettig oder verzweigt), Phenyl, wobei die bezüglich R(5) genannten Phenylsysteme unsubstituiert oder ein- bis 2-fach substituiert sind durch Substituenten aus der Gruppe F, Cl, $(C_1\text{-}C_4)$-Alkyl (geradkettig oder verzweigt), $(C_3\text{-}C_6)$-Cycloalkyl, $(C_1\text{-}C_4)$-Alkoxy (geradkettig oder verzweigt), Benzyl, Phenethyl,
und

X Sauerstoff oder Schwefel.

Europäisches Patentamt

# EUROPÄISCHER RECHERCHENBERICHT

Nummer der Anmeldung

EP 91 12 0186

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 237 917 (HOECHST)<br>* Ansprüche 1-7 *<br>--- | 1-9 | C07C323/30<br>C07C217/52<br>C07C217/70<br>C07D295/08<br>C07D261/08<br>C07D213/32<br>C07D211/58<br>C07D333/28<br>C07D209/16<br>A61K31/135<br>A61K31/33<br>A01N33/04<br>A01N43/00 |
| A | EP-A-0 313 782 (BAYER)<br>----- | | |
| | | | **RECHERCHIERTE SACHGEBIETE (Int. Cl.5 )** |
| | | | C07C<br>C07D |

Der vorliegende Recherchenbericht wurde für alle Patentansprüche erstellt

| Recherchenort | Abschlußdatum der Recherche | Prüfer |
|---|---|---|
| DEN HAAG | 31 JANUAR 1992 | ZAROKOSTAS K. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument